# EUROPEAN PATENT SPECIFICATION

(11) **EP 0 691 966 B1**
(45) Date of publication and mention of the grant of the patent: **09.09.1998**
(21) Application number: 94911662.8
(22) Date of filing: 23.03.1994
(51) Int. Cl.: C07D 401/06, A61K 31/47, C07D 215/06, C07D 215/12, C07D 215/14, C07D 405/04, C07D 401/04

(54) **QUINOLINES AS TYPE IV PHOSPHODIESTERASE INHIBITORS**
CHINOLINE ALS TYP IV PHOSPHODIESTERASE INHIBITOREN
QUINOLEINES COMME INHIBITEURS DE LA PHOSPHODIESTERASE TYPE IV

(30) Priority: 31.03.1993 US 40731
(43) Date of publication of application: 17.01.1996
(73) Proprietor: SYNTEX (U.S.A.) INC., Palo Alto California 94304 (US)
(72) Inventor: WILHELM, Robert, Stephen, Mountain View, CA 94040 (US); FATHEREE, Paul, Ross, San Francisco, CA 94117 (US); CHIN, Ronnie, Lipp, Mountain View, CA 94043 (US)
(74) Representative: Witte, Hubert, Dr.
(86) International application number: PCT/US94/03004
(87) International publication number: WO 94/22852

(56) References cited:
- CHEMICAL ABSTRACTS, vol. 111, no. 25, 18 December 1989, Columbus, Ohio, US; abstract no. 232740e, O.W. DAVID ET AL. 'Heterocyclic mesomeric betaines. Part 2. Synthesis ...' & J. CHEM. SOC., PERKIN TRANS. 1 1989, (5), 953-6
- JOURNAL OF HETEROCYCLIC CHEMISTRY August 1975 , PROVO US pages 703 - 704 P. HASSANALY ET AL. 'Homolytic aromatic substitution ...'
- CHEMICAL ABSTRACTS, vol. 71, no. 1, 7 July 1969, Columbus, Ohio, US; abstract no. 3230b, J.B. WOMMACK ET AL. 'Synthesis of quinoline- and isoquinolinecarboxaldehydes.' & J. HETEROCYCL. CHEM. 1969, 6 (2), 243-5
- CHEMICAL ABSTRACTS, vol. 93, no. 23, 8 December 1980, Columbus, Ohio, US; abstract no. 220557x, M. HOENEL ET AL. 'Selectivity in the hydrogenation of 6- and 8-substituted quinolines.' & J. CHEM. SOC., PERKIN TRANS. 1 1980, (9), 1933-9
- JOURNAL OF MEDICINAL CHEMISTRY vol. 34, no. 2 , 1991 , WASHINGTON US pages 624 - 628 JH.A. LOWE ET AL. 'Structure-activity relationship of quinazolinedione inhibitors of calcium-independent phosphodiesterase'
- Journal of the American Chemical Society,(68),1946,p.2589- 2592.

## Description

### BACKGROUND OF THE INVENTION

### FIELD OF THE INVENTION

This invention relates to optionally 6,8-substituted quinolines useful as anti-inflammatory agents, immunosuppressive agents, anti-allograft rejection agents, anti-graft-vs-host disease agents, anti-allergic agents (e.g., asthma, rhinitis and atopic dermatitis), bronchodilation agents, anti-autoimmune agents or analgetic agents, to their precursors, to their preparation and to pharmaceutical compositions using the compounds of the invention.

### BACKGROUND INFORMATION

Cyclic 3',5'-adenosine monophosphate (cAMP) modulates a variety of cellular and physiologic functions in mammals, such as, cell division, endocrine function, and the immune response. The level of cAMP is controlled by a class of enzymes called phosphodiesterases, which enzymatically deactivate cAMP. There are five general types of phosphodiesterases, which are categorized according to their function and the type of cell from which they are isolated. For instance, high-affinity phosphodiesterase (PDE III) is isolated from human platelet cells and modulates platelet aggregation. Another type of phosphodiesterase (PDE IV) is found in various tissues but is the predominant form in human leukocytes; this enzyme modulates leukocyte activation and function associated with the immune response and inflammation. Both of these phosphodiesterases implement their control by modulating the cellular level of cAMP in their respective cells. Thus, inhibition of phosphodiesterases provides a method of modulating any cellular and bodily function that is controlled by cAMP.

Compounds that are nonspecific phosphodiesterase inhibitors are known, i.e., these compounds inhibit all or multiple types of phosphodiesterases. [*See,* Beavo, J.A. and D.H. Reifsyder, *Trends in Pharm. Science,* 11:150-155 (1990); and Nicholson, C.D., R.A.J. Challiss and M. Shahid, *Trends in Pharm. Science,* 12:19-27 (1991).] Since cAMP is involved in so many functions throughout the body, a nonspecific phosphodiesterase inhibitor has the potential to alter all of the functions modulated by cAMP, thus nonspecific phosphodiesterase inhibitors are of limited value because of numerous side-effects.

It has been surprisingly discovered that certain optionally substituted 6,8-quinolines are potent selective inhibitors of Phosphodiesterase Type IV (PDE IV). These compounds are well suited for use as a treatment for any disorder in which PDE IV function plays a role, such as where leukocyte activation or function is involved.
In particular, these compounds are especially well suited for use as anti-inflammatory agents, immunosuppressive agents, anti-allograft rejection agents, anti-graft-vs-host disease agents, anti-allergic agents (e.g., asthma, rhinitis and atopic dermatitis), bronchodilation agents, anti-autoimmune disease agents or analgetic agents.

Certain 8-substituted quinolines are disclosed in the following documents: 8-(o-methoxyphenyl)-quinoline (Journal of Het. Chem., 1975, p. 703-704); 8-phenyl-6-quinolinecarboxaldehyde (Chem. Abs. , vol. 71, 1, 1969, no. 3230b); 8-phenylquinoline (Chem. Abs., vol. 93, 23, 1980, no.220557x); 6-methoxy-8-(p-nitrophenyl)-quinoline and 8-(p-aminophenyl)-6-methoxyquinoline (Journal of the Amer. Chem. Society, vol. 68, 1946, p. 2589-2592). The above documents do not disclose any biological activity data of the cited compounds.

### SUMMARY OF THE INVENTION

One aspect of the present invention relates to optionally substituted 6,8-quinolines, i.e., a compound of Formula I: 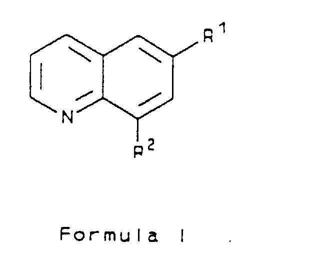 wherein:
R¹ is selected from lower alkyl; cycloalkyl; cycloalkyloxy; cycloalkylamino; cycloalkyl lower alkyl; lower alkoxy; hydroxy-lower alkyl; carboxyalkyl; optionally substituted aryl, aryloxy, arylamino or aryl lower alkyl; optionally substituted heterocycle, heterocycle-oxy, heterocycle-amino or heterocycle lower alkyl; and
R² is optionally substituted phenyl;
   provided that when R¹ is methoxy, R² is not 4-aminophenyl or 4-nitrophenyl;
   or a pharmaceutically acceptable salt or N-oxide thereof.

Preferred aspects of R¹ are pyridylmethyl, benzyl, cycloalkylmethyl and lower alkyl.

In another aspect, the invention relates to a pharmaceutical composition containing a therapeutically effective amount of a compound of Formula I or a pharmaceutically acceptable salt or N-oxide thereof admixed with at least one pharmaceutically acceptable excipient.

In still another aspect, the invention relates to a compound represented by the formula I or pharmaceutically acceptable salt or N-oxide thereof wherein R¹ and R² are defined as above and additionally wherein R¹ can be hydrogen or formyl for use as an anti-inflammatory agent, immunosuppressive agent, anti-allograft rejection agent, anti-graft-vs-host disease agent, anti-allergic agent (e.g., asthma, rhinitis and atropic dermatitis), bronchodilatation agents, anti-autoimmune disease agent or analgetic agent, by administering to a mammal in need of such treatment a therapeutically effective amount of a compound as above or a pharmaceutically acceptable salt or N-oxide thereof.

Yet another aspect of the invention relates to the treatment of the above conditions or diseases by the selective inhibition of PDE IV.

In another aspect, this invention provides compositions useful in the treatment of inflammatory, allograft rejection, graft-vs-host disease, allergy, autoimmune or analgetic conditions or diseases in mammals comprising a therapeutically effective amount of a compound of Formula I or a pharmaceutically acceptable salt or N-oxide as described above and a pharmaceutically acceptable excipient.

Another aspect of the invention relates to processes for making the compounds of Formula I and the pharmaceutically acceptable salts and N-oxides thereof.

### DETAILED DESCRIPTION

### DEFINITIONS AND GENERAL PARAMETERS

The following definitions are set forth to illustrate and define the meaning and scope of the various terms used to describe the invention herein.

The term "alkyl" refers to a branched or straight chain monovalent saturated aliphatic hydrocarbon radical of one to twenty carbon atoms.

The term "lower alkyl" refers to a branched or straight chain monovalent alkyl radical of one to four carbon atoms. This term is further exemplified by such radicals as methyl, ethyl, *n*-propyl, isopropyl, *i*-butyl, *n*-butyl and *t*-butyl.

The term "cycloalkyl" refers to a monovalent carbocyclic radical of three to six carbon atoms, i.e., cyclopropyl, cyclobutyl, cyclopentyl and cyclohexyl, which can optionally be substituted, independently, with, e.g., hydroxy, amino, imino, lower alkyl, lower alkoxy, carboxy, lower alkoxycarbonyl, carbamoyl, acyl, aryl, halo, and/or cyano.

The term "cycloalkyloxy" refers to a cycloalkyl group attached to a parent structure via an oxy radical, i.e., -O-, such as cyclopentyloxy.

The term "cycloalkylamino" refers to a cycloalkyl group attached to a parent structure via an imino radical, i.e., -NH-, such as cyclopropylamino.

The term "cycloalkyl lower alkyl" refers to a cycloalkyl group attached to a parent structure via a lower alkylene group such as methylene; e.g., cyclopropylmethyl, cyclopentylethyl, cyclopentylpropyl, or cyclopentylmethyl.

The term "lower alkylene" refers to a biradical branched or unbranched saturated hydrocarbon chain containing 1 to 4 carbon atoms, such as methylene (-CH₂-), ethylene, propylene, isopropylene and butylene.

The term "lower alkoxy" refers to the group -O-R' where R' is lower alkyl.

The term "carbonyl" refers to the group -C(O)-.

The term "carboxy" refers to the group -C(O)OH.

The term "carboxyalkyl" refers to the group -alkyl-C(O)OH,
where alkyl is a branched or straight chain monovalent alkyl radical of one to eight carbon atoms.

The term "lower alkoxycarbonyl" refers to the group -C(O)OR' where R' is lower alkyl.

The term "acyl" refers to the group -C(O)-R', where R' is lower alkyl, e.g., acetyl or propionyl; or optionally substituted phenyl or heterocycle, e.g., phenacyl.

The term "carbamoyl" refers to the group -C(O)NR'R where R and R' are independently hydrogen or lower alkyl, e.g., where R is hydrogen and R' is lower-alkyl the group is lower alkylcarbamoyl, where R and R' are lower alkyl the group is di-lower alkylcarbamoyl.

The term "halo" refers to fluoro, bromo, chloro and iodo.

The term "lower alkylthio" refers to the group R-S-.

The term "lower alkylsulfinyl" refers to the group R-S(O)-.

The term "lower alkylsulfonyl" refers to the group R-S(O)₂-.

The term "lower alkoxysulfonyl" refers to the group RO-S(O)₂-.

The term "hydroxysulfonyl" refers to the group HO-S(O₂)-.

The term "aryl" refers to a monovalent carbocyclic aromatic radical (e.g., phenyl), or two condensed carbocyclic rings (e.g., naphthyl) which can optionally be mono-, di-, or tri-substituted, independently, with hydroxy, thiol, amino, halo, nitro, lower alkyl, lower alkylthio, lower alkoxy, mono-lower alkylamino, di-lower alkylamino, carboxy, lower alkoxycarbonyl, hydroxysulfonyl, lower alkoxysulfonyl, lower alkylsulfonyl, lower alkylsulfinyl, trifluoromethyl, cyano, tetrazolyl, carbamoyl, lower alkylcarbamoyl, and di-lower alkylcarbamoyl.

The term "aryloxy" refers to an aryl group as defined above attached to a parent structure via an oxy radical, i.e., aryl-O-.

The term "arylamino" refers to an aryl group as defined above attached to a parent structure via an imino radical, i.e., aryl-NH-.

The term "aryl lower alkyl" refers to an aryl group attached to a parent structure via a lower alkylene group such as methylene; e.g., benzyl.

The term "heterocycle" refers to a saturated, unsaturated or aromatic monovalent cyclic radical having at least one hetero atom (such as nitrogen, oxygen or sulfur) or a combination thereof, which can optionally be substituted, independently, with, e.g., hydroxy, amino, imino, lower alkyl, lower alkoxy, carboxy, lower alkoxycarbonyl, carbamoyl, acyl, aryl, halo, and/or cyano. Further, the term also includes instances where an atom of a heterocycle has been oxidized, e.g., N-oxides, sulfoxides, sulfones, or oxo. For example, typical heterocycles with one or more nitrogen or sulfur atoms are pyrrole, imidazole, imidazoline, imidazolidine, pyrazole, pyrazine, pyrrolidine, pyrrolidinone, pyrazolidine, piperidine, piperazine, morpholine, pyridine, pyridone, triazole, oxazole, oxadiazole, thiazole, and the like.

The term "heterocycle-oxy" refers to a heterocyclic group as defined above attached to a parent structure via an oxy radical, i.e., heterocycle-O-.

The term "heterocycle-amino" refers to a heterocyclic group as defined above attached to a parent structure via an imino radical, i.e., heterocycle-NH-.

The term "heterocycle lower alkyl" refers to a heterocyclic group attached to a parent structure via a lower alkylene group such as methylene (-CH₂-) ; e.g., 4-pyridylmethyl.

The term "tetrazolyl" refers to the group

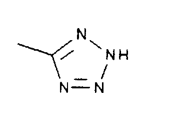

The term "optionally substituted phenyl" refers to phenyl and mono-, di-, tri- or tetra-substituted phenyl, wherein the optional substituents are lower alkyl, hydroxy, thiol, amino, halo, nitro, cyano, lower alkoxy, lower alkylthio, mono-lower alkylamino, di-lower-alkylamino, carboxy, lower alkoxycarbonyl, lower alkylene-dioxy, hydroxysulfonyl, lower alkoxysulfonyl, lower alkylsulfonyl, lower-alkylsulfinyl, trifluoromethyl, trifluoromethyloxy, tetrazolyl, carbamoyl, lower alkylcarbamoyl, and di-lower alkylcarbamoyl. This term is further exemplified by such radicals as 3-chlorophenyl, 3-nitrophenyl, 4-methoxyphenyl, 3-cyanophenyl, 4-trifluorophenyl, 3-chloro-4-fluorophenyl and 3,4-methylenedioxyphenyl.

The term "N-oxide" refers to nitrogen heterocycles where a nitrogen atom in the ring has been oxidized, e.g., 4-pyridyl-N-oxide, 3-pyridyl-N-oxide, or 2-pyridyl-N-oxide.

The term "hydroxy-lower alkyl" refers to a lower alkyl radical substituted with a hydroxy group, e.g., -CH(OH)CH₂CH₃ or -C(OH)(CH₃)₂.

The term "pharmaceutically acceptable salt" refers to any salt derived from an inorganic or organic acid or base.

The term "pharmaceutically acceptable anion" refers to the anion of such acid addition salts. The term "pharmaceutically acceptable cation" refers to the cation of such base addition salts. The salt, anion and/or the cation are chosen not to be biologically or otherwise undesirable. The anions are derived from inorganic acids, such as hydrochloric acid, hydrobromic acid, sulfuric acid (giving the sulfate and bisulfate salts), nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, salicylic acid, p-toluenesulfonic acid and the like.
The cations are derived from bases, such as alkaline earth hydroxides, including calcium hydroxide, potassium hydroxide, sodium hydroxide, lithium hydroxide and the like.

As used herein, the term "allograft rejection" refers to the humoral or cellular immune response mounted by the immune system of a mammal after it has received a histo-incompatible tissue graft from another mammal of the same species, thereby producing tissue injury to the graft in such a recipient.

As used herein, the term "graft-vs-host disease" refers to the immune response that originates from transplanted graft tissue, in particular, transplanted bone-marrow tissue, and that is directed towards the host tissue, thereby producing tissue injury in the host.

As used herein, the term "autoimmune disease" refers to disorders wherein the immune system of a mammal mounts a humoral or cellular immune response to the mammal's own tissue or to antigenic agents that are not intrinsically harmful to the mammal, thereby producing tissue injury in such a mammal. Examples of such disorders include, but are not limited to, systemic lupus erythematosus, rheumatoid arthritis and type I diabetes.

As used herein, the terms "treatment" or "treating" of a condition and/or a disease in a mammal, means:
(i) preventing the condition or disease, that is, avoiding any clinical symptoms of the disease;
(ii) inhibiting the condition or disease, that is, arresting the development or progression of clinical symptoms; and/or
(iii) relieving the condition or disease, that is, causing the regression of clinical symptoms.

The conditions and diseases treated in the present invention include inflammation, pain, pyrexia, autoimmune disease, allograft rejection, graft-vs-host disease, allergies and uveitis.

As used herein, the term "therapeutically effective amount" refers to that amount of a compound of formula I wherein R¹ and R² are defined as above and additionally wherein R¹ can be hydrogen or formyl which, when administered to a mammal in need thereof, is sufficient to effect treatment (as defined above) as an anti-inflammatory agent, immunosuppressive agent, anti-allograft rejection agent, anti-graft-vs-host disease agent, anti-allergy agent, autoimmune disease agent or analgetic agent. The amount that constitutes a "therapeutically effective amount" will vary depending on the compound, the condition or disease and its severity, and the mammal to be treated, but may be determined routinely by one of ordinary skill in the art with regard to contemporary knowledge and to this disclosure.

As used herein, the term "q.s." means adding a quantity sufficient to achieve a stated function, e.g., to bring a solution to a desired volume (e.g., 100 mL).

As used herein, the term "mp" refers to melting point
All temperatures are given in degrees Celsius (°C).

Unless specified to the contrary, the reactions described herein take place at atmospheric pressure over a temperature range from about -78°C to about 150°C, more preferably from about 10°C to about 50°C, and most preferably at about room (or "ambient") temperature, e.g., about 20°C. Unless specified to the contrary, the ranges of time and temperature described herein are approximate, e.g., "from 8 to 24 hours at from 10°C to 100°C" means from about 8 to about 24 hours at about 10°C to about 100°C.

Isolation and purification of the compounds and intermediates described herein can be effected, if desired, by any suitable separation or purification procedure such as, for example, filtration, extraction, crystallization, column chromatography, preparative high pressure liquid chromatography (preparative HPLC), thin-layer chromatography or thick-layer chromatography, or a combination of these procedures. Specific illustrations of suitable separation and isolation procedures can be had by reference to the examples hereinbelow. However, other equivalent separation or isolation procedures can also be used.

The following numbering and nomenclature system will be used for naming the compounds of the invention.

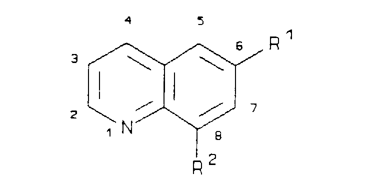

E.g., the compound of Formula I where R¹ is 4-pyridylmethyl and R² is 3-chloro-4-fluorophenyl can be named 6-(4-pyridylmethyl)-8-(3-chloro-4-fluorophenyl)quinoline.

The compound of Formula I where R¹ is isopropyl and R² is 4-chlorophenyl can be named 6-(isopropyl)-8-(4-chlorophenyl)quinoline.

### SYNTHESIS OF THE COMPOUNDS OF FORMULA I

As used in the Reaction Schemes R¹ and R² are the same as described in the Summary of the Invention.

Reaction Scheme A illustrates the preparation of 6,8-(disubstituted) quinolines, i.e., the compounds of Formula I.

Reaction Scheme A-l illustrates the preparation of intermediates of formula 3a which can be converted into compounds of formula I where R¹ is cycloalkylmethyl, arylmethyl or heterocycle-methyl by following the reactions as shown in Reaction Scheme A.

Reaction Scheme B illustrates an alternate preparation of 6,8-(disubstituted) quinolines, i.e., the compounds of Formula I, where the final two steps in Reaction Scheme A are carried out in reverse order.

Reaction Scheme B-1 illustrates an alternate preparation of 6,8-(disubstituted) quinolines, i.e., the compounds of Formula I, via the 8-znCl-quinoline intermediate.

Reaction Scheme B-2 illustrates the preparation of 6-substituted-8-haloquinolines, in particular, the compounds of Formula 5 where X is halo and R¹ is cycloalkylaminomethyl, arylaminomethyl, heterocycle-aminomethyl or heterocycle-methyl, i.e., where the methylene group is attached to a nitrogen atom of a heterocycle.

Reaction Scheme B-3 illustrates the preparation of 6,8-(disubstituted) quinolines, in particular, the compounds of Formula I where R¹ is cycloalkyloxy, aryloxy or heterocycle-oxy.

Reaction Scheme C illustrates the preparation of 6- [lower alkyl- (hydroxy)methyl] -8-quinolines, and 6-formyl-8-quinolines, i.e., the compounds of Formula I where R¹ is formyl, or lower alkyl-(hydroxy)methyl.

Reaction Schemes C-1 and C-2 illustrate alternate preparations of 6,8-(disubstituted)quinolines, in particular, the compounds of Formula I where R¹ is alkyl, cycloalkylmethyl, cycloalkenylmethyl, arylmethyl or heterocycle-methyl.

Reaction Scheme C-3 illustrates an alternate preparation of 6,8-(disubstituted)quinolines, in particular, the compounds of Formula I where R¹ is mono- or di-alkylaminomethyl, cycloalkylaminomethyl, arylaminomethyl, heterocycle-aminomethyl or heterocycle-methyl, i.e., where the methylene group is attached to a nitrogen atom of a heterocycle.

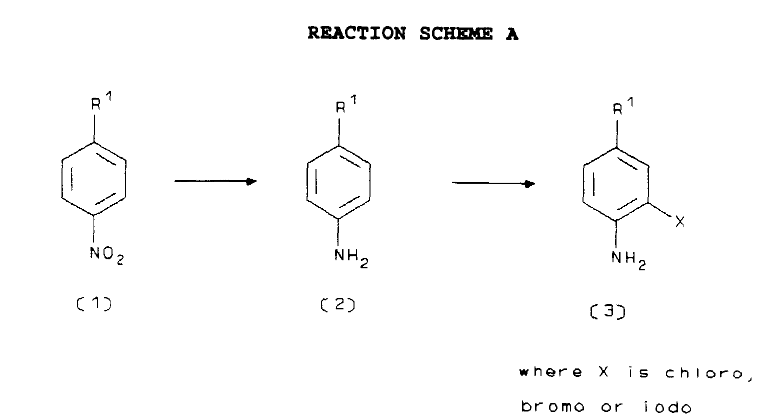

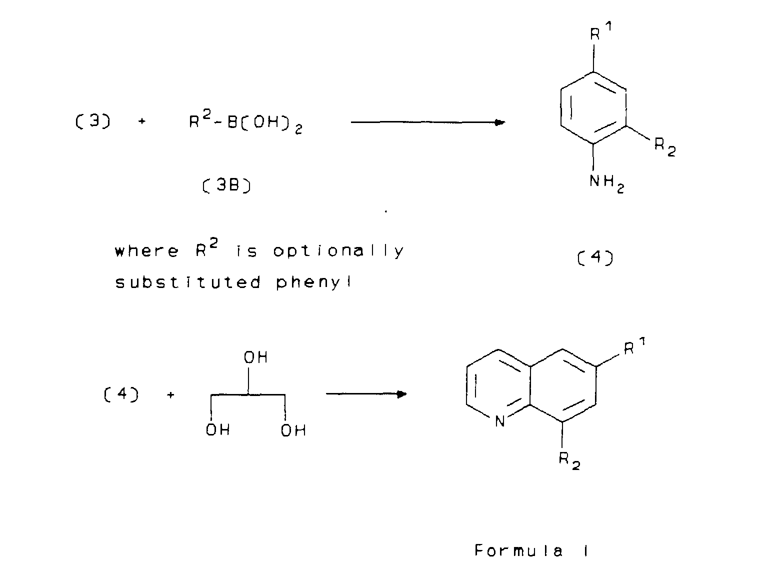

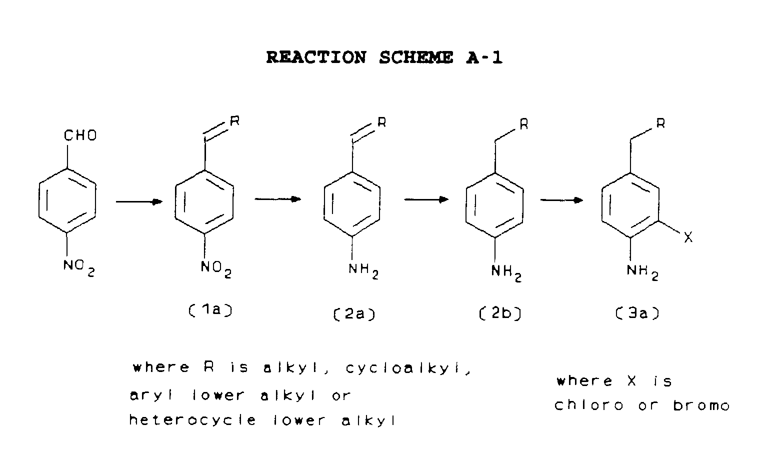

### STARTING MATERIALS

Referring to the Reaction Schemes, the compounds of Formula 1 (i.e., 4-optionally substituted nitrobenzene) and Formula 2 (i.e., 4-optionally substituted aniline) are commercially available from Aldrich Chemicals Co., Inc., Fluka Chemical Corporation, Lancaster Synthesis Ltd., Karl Industries, Maybridge Chemical Co. Ltd. or Tokyo Kasai International. The compounds of Formula 3A, i.e., optionally substituted benzene boronic acid, are commercially available from Lancaster Synthesis Ltd., or alternatively can be prepared following the procedures in Organic Synthesis, Coll Vol 4. Those compounds that are not commercially available can be prepared by one of ordinary skill in the art following procedures set forth in references such as, "Fieser and Fieser's Reagents for Organic Synthesis", Volumes 1-15, John Wiley and Sons, 1991; "Rodd's Chemistry of Carbon Compounds", Volumes 1-5 and Supplementals, Elservier Science Publishers, 1989; and "Organic Reactions", Volumes 1-40, John Wiley and Sons, 1991.

### PREPARATION OF FORMULA 2

An optionally substituted p-nitrobenzene is combined with about 5 molar equivalents of a reducing agent, such as SnCl₂·H₂O, Fe/acetic acid, or palladium on carbon/H₂, preferably SnCl₂·H₂O, in a solvent such as ethanol, or ethyl acetate, preferably ethanol. The solution is heated. The temperature and duration will vary according to the reagent and solvent used, e.g., using ethanol and Sncl₂·H₂O, the solution is heated at a temperature in the range of about 50°C to 90°C, preferably about 70°C, for a period of about 1 hour to 3 hours, preferably about 2 hours. The progress of the reaction is monitored by TLC (thin layer chromatography). When the reaction is substantially complete, the product is isolated and purified by conventional means yielding the desired optionally substituted p-aminobenzene compound (i.e., a compound of Formula 2).

### PREPARATION OF FORMULA 3

A solution of about 1 molar equivalent of a halogenating agent, such as N-bromosuccinimide (NBS), or N-chlorosuccinimide (NCS), preferably N-bromosuccinimide, in a solvent, preferably DMF is added in a gradual manner to a solution of an optionally substituted p-aminobenzene compound (Formula 2) dissolved in a solvent (such as DMF, preferably DMF). The reaction mixture is stirred at about room temperature for a period of about 1 to 5 hours, preferably about 3 hours. When the reaction is substantially complete (by TLC), the product is isolated and purified by conventional means yielding the desired optionally substituted *p*-amino-*m*-halobenzene compound (i.e., a compound of Formula 3 where X is chloro or bromo).

### PREPARATION OF FORMULA 4

An optionally substituted *p*-amino-*m*-halobenzene compound (Formula 3) is combined with about 1 to 5 molar equivalent, preferably about 2 molar equivalent, of an optionally substituted benzene boronic acid, i.e., Formula 3B where R² is optionally substituted phenyl, 2 M Na₂CO₃ (about 4 molar equivalent), methanol or ethanol, preferably ethanol, and benzene or toluene, preferably benzene. To this solution is added about 0.01 to 0.1 molar equivalent, preferably about 0.035 molar equivalent, of palladium tetrakis triphenylphosphine. The reaction mixture is heated at about reflux for a period of about 3 to 9 hours, preferably about 6 hours. When the reaction is substantially complete (by TLC), the product is isolated and purified by conventional means yielding the desired optionally substituted *p*-amino-*m*-arylbenzene compound (e.g., a compound of Formula 4)

### PREPARATION OF FORMULA I

An optionally substituted *p*-amino-*m*-arylbenzene compound (Formula 4) is combined with about 1 molar equivalent of an oxidant, such as ferric oxide, *m*-nitrobenzenesulphonic acid, nitrobenzene, iron(II) sulfate (heptahydrate)/nitrobenzene or arsenic pentoxide, preferably arsenic pentoxide, and 3 molar equivalent of glycerol under an inert atmosphere. The mixture is heated at a temperature in the range of about 75°C to 125°C, preferably about 100°C, for a period of about 15 minutes to 45 minutes, preferably about 30 minutes. About 12 molar equivalent of a concentrated acid (preferably concentrated H₂SO₄) is added to the mixture in a gradual manner, and the mixture is heated at a temperature in the range of about 100°C to 200°C, preferably about 150°C for a period of about 1 to 3 hours, preferably about 2 hours. The progress of the reaction is monitored by TLC (9:1 hexane:ethyl acetate). When the reaction is substantially complete (by TLC), the product is isolated and purified by conventional means yielding the desired 6-optionally substituted-8-arylquinoline compound (i.e., a compound of Formula I).

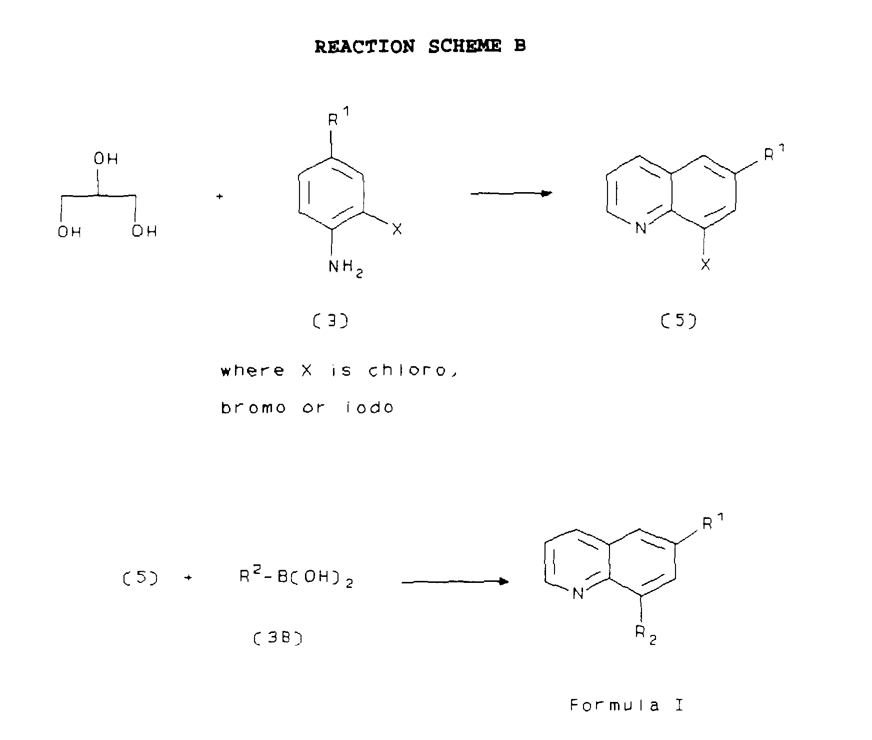

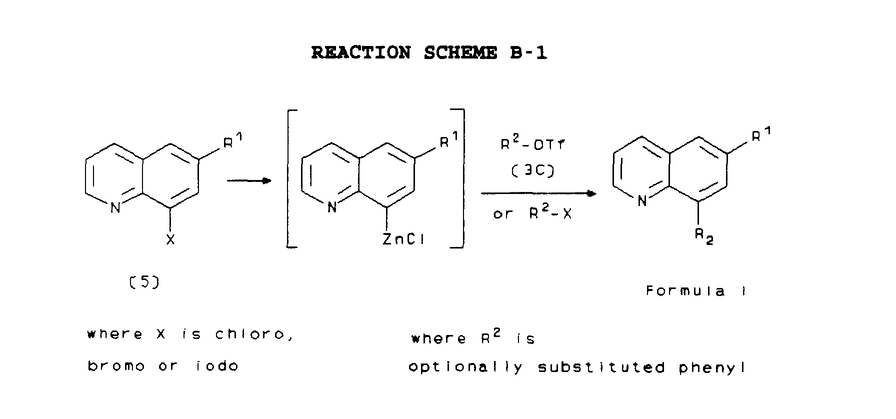

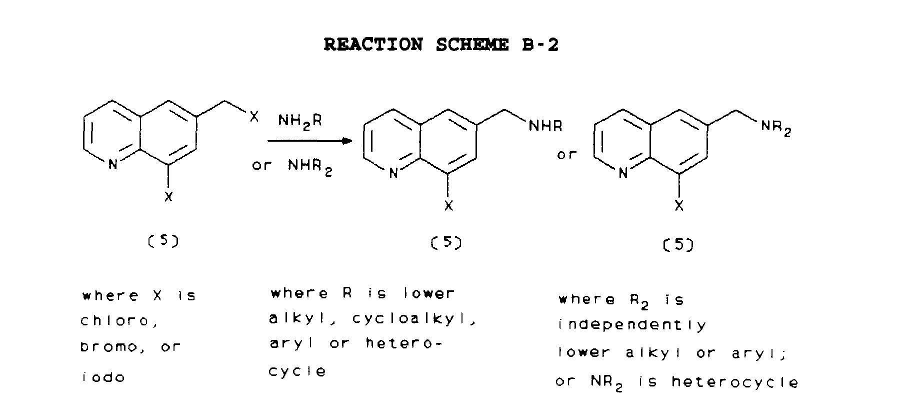

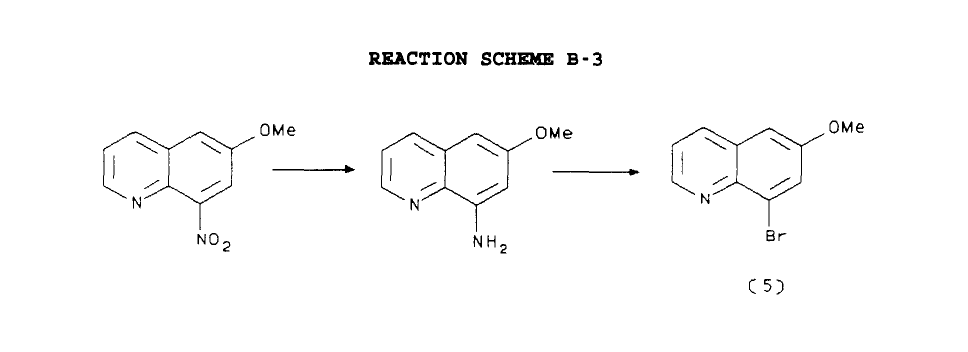

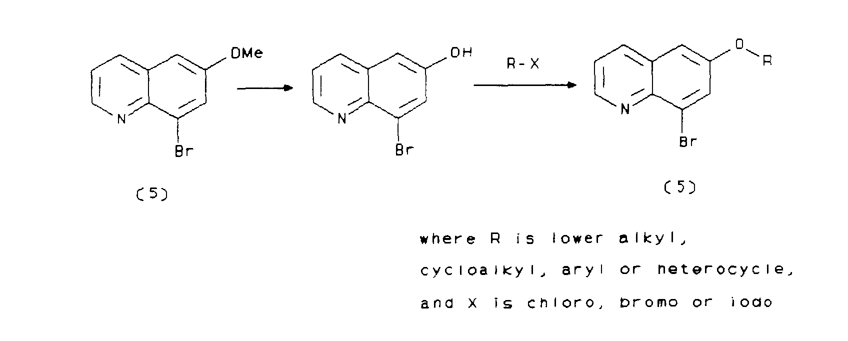

### PREPARATION OF FORMULA 5

An optionally substituted *p*-amino-*m*-halobenzene compound (Formula 3) combined with about 1 molar equivalent of arsenic pentoxide and 3 molar equivalent of glycerol under an inert atmosphere. The mixture is heated at a temperature in the range of about 75°C to 125°C, preferably about 100°C, for a period of about 15 minutes to 45 minutes, preferably about 30 minutes. About 12 molar equivalent of a concentrated acid (preferably concentrated H₂SO₄) is added to the mixture in a gradual manner, and the mixture is heated at a temperature in the range of about 100°C to 200°C, preferably about 150°C for a period of about 1 to 3 hours, preferably about 2 hours. When the reaction is substantially complete (by TLC), the product is isolated and purified by conventional means yielding the desired 6-optionally substituted-8-haloquinoline compound (i.e., a compound of Formula 5).

### PREPARATION OF FORMULA 3B

About 2 molar equivalents of trimethylborate is dissolved in an apolar solvent (such as diethyl ether, or tetrahydrofuran, preferably diethyl ether) and cooled at a temperature in the range of about -50 to -80°C, preferably about -65°C.
An optionally substituted phenyl Grignard reagent is added to the solution in a gradual (e.g., dropwise) manner over a period of about 20 minutes/molar equivalent. The mixture is then stirred at a temperature in the range of about -50°C to -80°C for about 15 to 45 minutes, preferably about 30 minutes. The mixture is allowed to warm to about -10°C to 10°C, preferably about 0°C and stirred for a period of about 1 hour. Water is added to the reaction mixture and the mixture is stirred for a period of about 1 hour. The organic layer is removed and the residue is extracted (e.g., 3 x ethyl acetate).
The organic layers are combined and dried over a drying agent (e.g., MgSO₄). The solution is concentrated, hexanes are added to the solution and the solution is stirred for a period of about 1 hour until a free flowing suspension forms. The suspension is filtered and air dried. The resultant optionally substituted benzene boronic acid (i.e., a compound of Formula 3B) is carried on to the next step of the process.

### PREPARATION OF FORMULA 3C

An optionally substituted phenol is dissolved in an aprotic solvent (such as CH₂Cl₂ or THF, preferably CH₂Cl₂) and cooled to 0 C. About 5 molar equivalents of triethylamine are added, followed by about 1.5 molar equivalents of trifluoromethanesulfonic anhydride (triflic anhydride) (e.g., via addition funnel) under nitrogen over a period of 15 min to 60 min, preferably 30 min. The solution is poured into a saturated NaHCO₃ solution and extracted with an aprotic solvent such as CH₂Cl₂. The organic layer is washed with water (2 x), and brine (100 mL), dried over a drying agent (e.g., MgSO₄), filtered, and concentrated. Flash chromatography using 10-50%, preferably 20%, ethyl acetate/hexanes provides a slightly impure brown oil which can be distilled under vacuum to give the trifluoromethanesulfonyloxy derivative (triflate) as a yellow liquid.

### PREPARATION OF FORMULA I

An 6-optionally substituted-8-haloquinoline compound (i.e., a compound of Formula 5) is combined with about 2 molar equivalent of an optionally substituted benzene boronic acid, i.e., Formula 3A, 2 M Na₂CO₃ (about 4 molar equivalent), methanol, and benzene. To this solution is added about 0.1 molar equivalent of palladium tetrakis triphenylphosphine. The reaction mixture is heated at about reflux for a period of about 3 to 9 hours, preferably about 6 hours. The progress of the reaction is monitored by TLC. Upon completion of the reaction, the mixture is allowed to cool to about room temperature, and the solvents are removed. About 40 mL/molar equivalent of ethyl acetate is added to the residue and filtered through a drying agent (e.g., Na₂SO₄). The product is isolated by chromatography, preferably preparative thin layer chromatography, yielding the desired 6-optionally substituted-8-aryl-quinoline compound (i.e., a compound of Formula I).

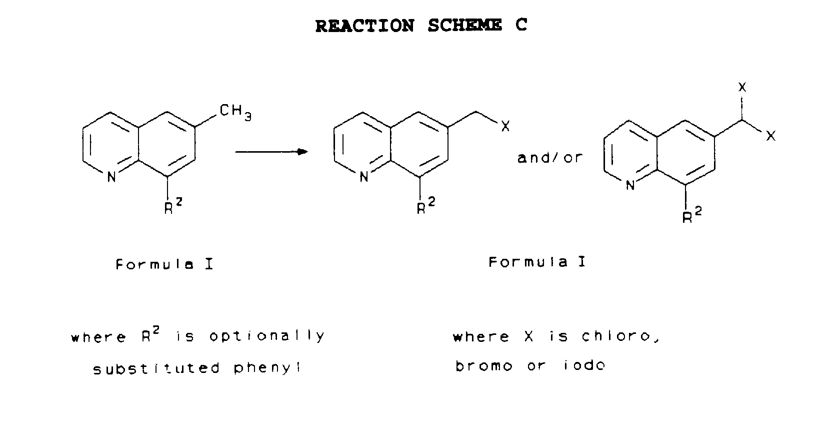

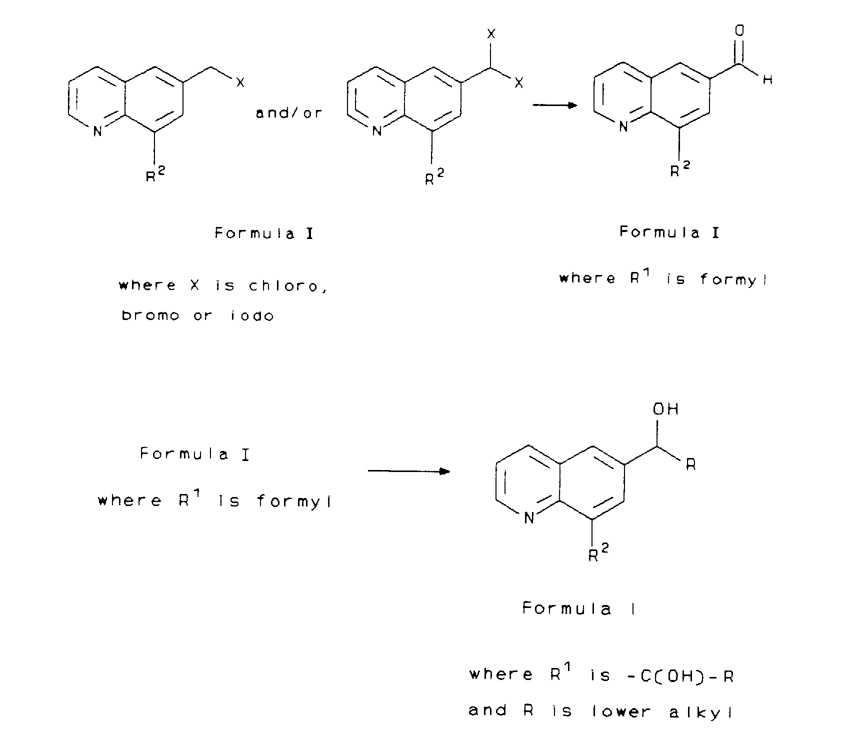

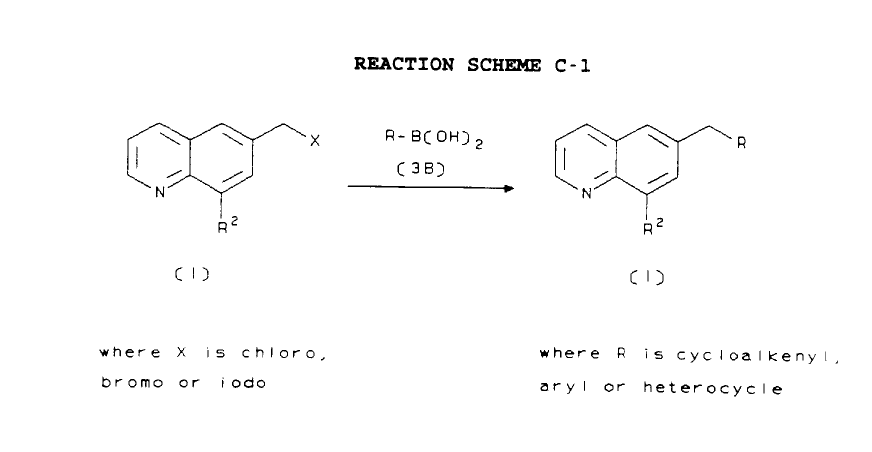

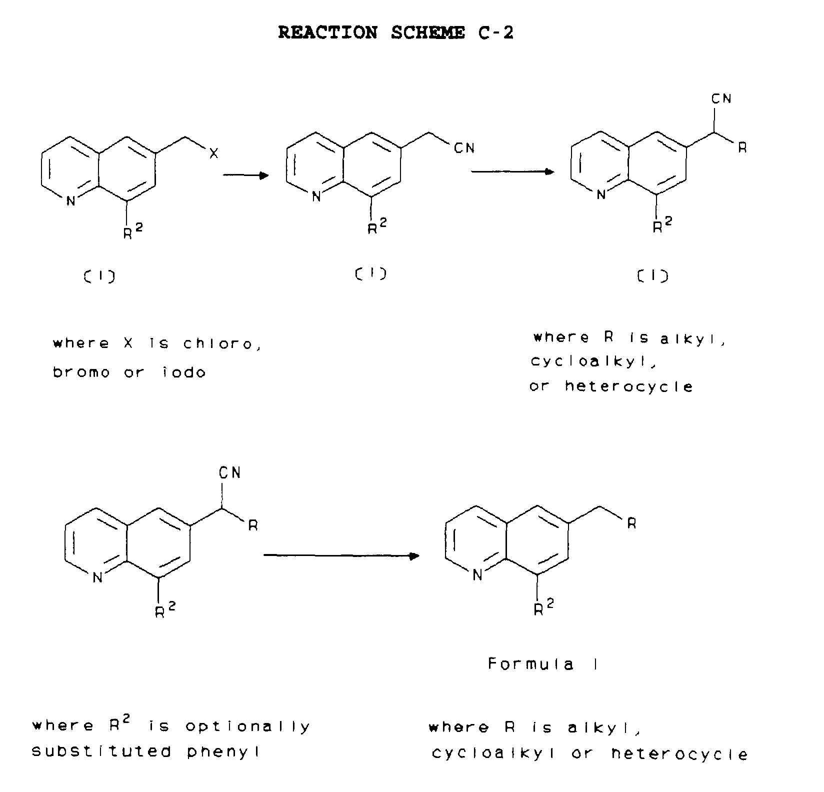

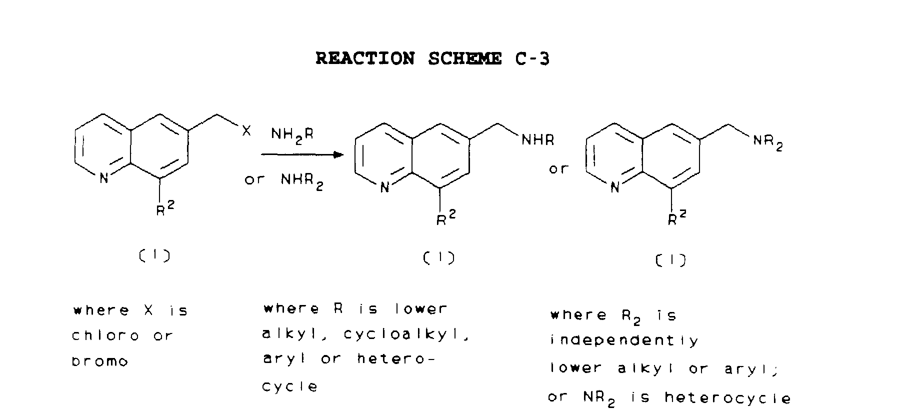

### PREPARATION OF FORMULA I WHERE R¹ IS CH₂-X AND/OR -CH-X₂

A 6-methyl-8-optionally substituted phenyl-quinoline (i.e., a compound of Formula I where R¹ is methyl, prepared according procedures described in Reaction Scheme A or B) is dissolved in a solvent such as carbon tetrachloride and heated under reflux. About 1 molar equivalent of a halogenating reagent, such as N-bromosuccinamide (NBS) or N-chlorosuccinamide (NCS), preferably N-bromosuccinamide and about 0.2 molar equivalent of 2,2'-azobis(2-methylpropionitrile) are added to the refluxing solution. The reaction mixture is optionally exposed to light (e.g., 250 W light bulb) for a period of about 30 minutes to 90 minutes, preferably about 1 hour. The reaction mixture is then stirred for a period of about 1 to 3 hours, preferably about 2 hours. The progress of the reaction is monitored by TLC. Upon conversion of greater than about 90% of the starting material, the reaction solution is cooled to about 0°C, then poured through a drying agent, such as Na₂SO₄. The solution is concentrated yielding the mono- and di-halogenated products (i.e., Formula I, where R¹ is CH₂-X and -CH-X₂) which are taken on to the next step without further purification or isolation.

### PREPARATION OF FORMULA I WHERE R¹ IS FORMYL

The mono and dihalogenated quinoline mixture from the previous step is dissolved in a solvent, such as methylene chloride or chloroform, preferably chloroform, and added in a gradual manner to an oxidizing reagent, such as about 5 molar equivalents of tetra *n*-butylammonium dichromate in a solvent, such as methylene chloride or chloroform, preferably chloroform. The reaction mixture is heated, preferably at reflux temperature, and refluxed for about 2 to 6 hours, preferably about 4 hours. The reaction mixture is allowed to cooled to about room temperature and filtered, preferably through a silica gel pad.
The residue is eluted with an ether, preferably diethyl ether, and isolated and purified by chromatography to obtain the desired 6-formyl-8-(optionally substituted phenyl)quinoline (i.e., Formula I where R¹ if formyl). Alternatively, the mono and di-halogenated quinoline mixture can be oxidized by the Kornblum oxidation [*Chem Rev,* Vol 67, No. 3, (1967), p 247-260] or the Sommelet oxidation [*Org. React.,* Vol 8, p 197-217 (1954)].

### PREPARATION OF FORMULA I WHERE R¹ IS -CH(OH) - (LOWER ALKYL)

A 6-formyl-8-(optionally substituted phenyl)quinoline is dissolved in a nonpolar solvent, preferably tetrahydrofuran, and cooled to a temperature in the range of about -50°C to -100°C, preferably about -78°C. To this solution is added an alkylation reagent, such as a lower-alkyl Grignard reagent, or a lower-alkyl lithium reagent (about 1 to 3 molar equivalent, preferably about 2 molar equivalent) in a gradual manner. The reaction mixture is stirred for a period of about 10 to 30 minutes, preferably about 20 minutes. The reaction mixture is then quenched with an aqueous salt solution, preferably saturated ammonium chloride. The desired product is purified by extraction (preferably with ethyl acetate), and drying (preferably over MgSO₄) . The residue is further purified and isolated by chromatography to yield the desired optionally substituted 6-[(lower alkyl(hydroxy)methyl]-8-(optionally substituted phenyl)quinoline, i.e., Formula I where R¹ is lower alkyl (hydroxy)methyl.

### PREPARATION OF THE SALT OF FORMULA I

The pharmaceutically acceptable salts of Formula I are prepared by dissolving a compound of Formula I in a suitable solvent (such as methanol, dioxane or diethyl ether) adding 1 to 3 molar equivalents (preferably about two molar equivalent) of an appropriate acid (such as hydrogen chloride gas) or base (such as an alkaline earth hydroxide, e.g., lithium hydroxide, calcium hydroxide, potassium hydroxide, sodium hydroxide or the like; preferably sodium hydroxide) and stirring.
The salt is isolated by lyophilization or by precipitation, using techniques that will be apparent to those skilled in the art.

### PREPARATION OF N-OXIDES OF FORMULA I

A compound of Formula I, where R¹ is pyridylmethyl (e.g., 4-pyridylmethyl, 3-pyridylmethyl or 2-pyridylmethyl), is converted to the corresponding N-oxide derivative by treating it with an oxidizing agent (e.g., *m*-chloroperoxybenzoic acid) in a solvent (e.g., methylene chloride). The solution is stirred for a period of about 30 to 90 minutes, preferably about 60 minutes at a temperature in the range of about 0°C to 50°C, preferably about room temperature. Following the reaction, the desired product is isolated and purified by preparative thin-layer chromatography.

### PREFERRED COMPOUNDS

Presently preferred are the compounds of Formula I where R² is 3-chlorophenyl, 3-nitrophenyl, 3-cyanophenyl or 3-chloro-4-fluoro- phenyl.

Of the compounds where R² is 3-chlorophenyl, 3-nitrophenyl or 3-cyanophenyl most preferred are the compounds of Formula I where and R¹ is 4-pyridylmethyl, benzyl, ethyl, n-propyl, isopropyl, n-butyl or 1-hydroxy1-methylethyl.

Of the compounds where R² is 3-chloro-4-fluorophenyl most preferred are the compounds of Formula I where R¹ is 4-pyridylmethyl, benzyl, ethyl, n-propyl, isopropyl, n-butyl or 1-hydroxy-1-methylethyl.

### PREFERRED PROCESSES

A preferred process for making 6-optionally substituted 8-arylquinolines is combining the corresponding optionally substituted benzene boronic acid with the corresponding 6-optionally substituted quinoline.

Another preferred process for making 6-optionally substituted 8-arylquinolines is combining the corresponding optionally substituted *p*-amino-*m*-benzene with glycerol and arsenic pentoxide.

Other preferred processes for making 6-optionally substituted 8-arylquinolines are shown in Reaction Schemes C, C-1, C-2, and C-3.

### LAST STEPS

The compounds of this invention are prepared according to the following processes:

A process for the preparation of a compound of the formula 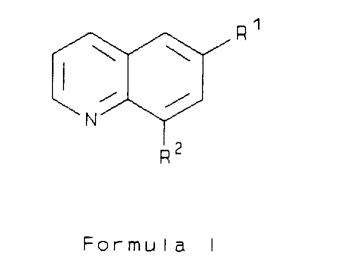 wherein:
- R¹: is selected from hydrogen; lower alkyl; cycloalkyl; cycloalkyloxy; cycloalkylamino; cycloalkyl lower alkyl; lower alkoxy; formyl; hydroxy-lower alkyl; carboxyalkyl; optionally substituted aryl, aryloxy, arylamino or aryl lower alkyl; optionally substituted heterocycle, heterocycle-oxy, heterocycle-amino or heterocycle lower alkyl; and
- R²: is optionally substituted phenyl,
provided that when R¹ is methoxy, R² is not 4-nitrophenyl or 4-aminophenyl;
or a pharmaceutically acceptable salt or N-oxide thereof, which comprises
(a) reacting a compound of the formula (4) 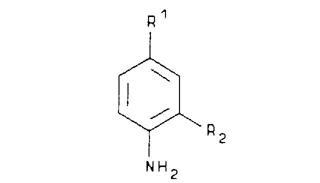 where
   R¹ and R² are as defined above, with glycerol in the presence of an oxidant; or
(b) reacting a compound of the formula (5) 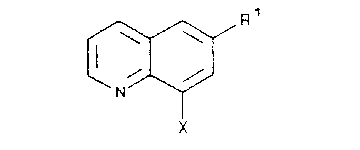 where
   R¹ is as defined above and X is chloro, bromo or iodo, with a boronic acid of the formula (3B)

   R²-B(OH)₂

   where R² is optionally substituted phenyl; or
(c) reacting a compound of the formula 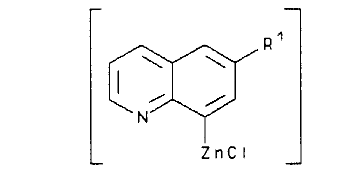 where
   R¹ is as defined above, with a trifluoromethanesulfonyloxy benzene of the formula (3C)

   R²-OTf

   where R² is optionally substituted phenyl, and -OTf is trifluoromethanesulfonyloxy; or
(d) reacting a compound of the formula (5) 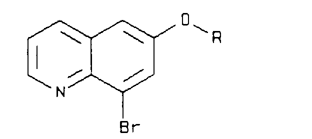 where
   R is lower alkyl, cycloalkyl, aryl, or heterocycle, with a boronic acid of the formula (3B)

   R²-B(OH)₂

   where R² is optionally substituted phenyl; or
(e) reacting a compound of the formula (I) 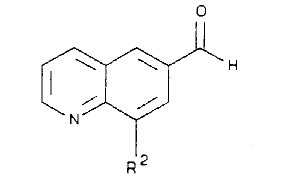 where
   R² is optionally substituted phenyl, with a lower alkyl Grignard reagent or a lower alkyl lithium reagent; or
(f) reacting a compound of the formula 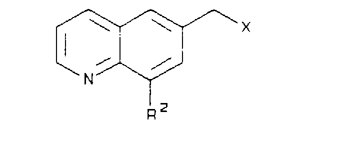 where
   R² is optionally substituted phenyl, with a boronic acid of the formula (3B)

   R-B(OH)₂

   where R is cycloalkenyl, aryl or heterocycle; or
(g) reacting a compound of the formula 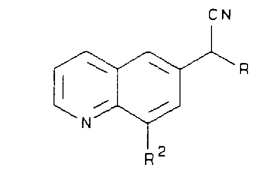 where
   R² is optionally substituted phenyl, and R is alkyl, cycloalkyl or heterocycle, with a concentrated acid; or
(h) reacting a compound of the formula 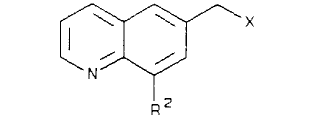 where
   R₂ is optionally substituted phenyl and X is chloro, bromo or iodo, with a compound of the formula

   NH₂R or NHR₂,

   where R is lower alkyl, cycloalkyl, aryl or heterocycle; and R₂ is independently lower alkyl or aryl; or NR₂ is heterocycle; or
(i) reacting the free base of a compound of Formula I with an acid to give a pharmaceutically acceptable acid addition salt; or
(j) reacting an acid addition salt of a compound of Formula I with a base to give the corresponding free base; or
(k) converting an acid addition salt of a compound of Formula I to another pharmaceutically acceptable acid addition salt of Formula I.

### UTILITY, TESTING AND ADMINISTRATION

### GENERAL UTILITY

The compounds of this invention, including the pharmaceutically acceptable salts and N-oxides thereof, and the compositions containing them are particularly useful as anti-inflammatory, immunosuppressive, anti-allograft rejection, anti-graft-vs-host disease, anti-allergic agents (e.g., asthma, rhinitis and atopic dermatitis), bronchodilation agents, anti-autoimmune disease or analgetic agents. The compounds of this invention act as PDE IV selective inhibitors, thereby modulating cAMP levels. Thus, these compounds are of use for the treatment of cAMP related conditions or diseases, particularly those that are modulated by leukocyte cAMP.

For example, inflammation, autoimmune diseases, graft-vs-host disease and allograft rejection are conditions that are manifested by the proliferation of lymphocytes. The proliferation is triggered by the presence of cAMP at specific levels. Inhibition of lymphocyte proliferation is accomplished by increasing levels of cAMP resulting from the inhibition of lymphocyte phosphodiesterase.

### TESTING

Potency and selectivity of compounds as inhibitors of PDE IV is determined by following, for example, the procedures described in Example 22, or modifications thereof.

The immunomodulatory and anti-inflammatory activity of the compounds of the invention can be determined by a variety of assays utilizing both *in vitro* and *in vivo* procedures.

Inhibition of the proliferation of lymphocytes in response to mitogenic stimulation is determined by the procedures described by Greaves, et al. ["Activation of human T and B lymphocytes by polyclonal mitogens," *Nature,* 248, 698-701 11974)], or modifications thereof (Example 23).

Inhibition of lymphocyte activation in response to antigenic challenge is determined *in vitro* by inhibition of a cytolytic T-cell assay (CTL) as described by Wunderlich, et al., *Nature* (1970), Vol. 228, p. 62, or a modification thereof.

Immune modulation is determined by *in vivo* procedures utilizing the Jerne Hemolytic Plaque Assay, [Jerne, et al., "The agar plaque technique for recognizing antibody producing cells," *Cell-bound Antibodies,* Amos, B. and Kaprowski, H. editors (Wistar Institute Press, Philadelphia) 1963, p. 109] or a modification thereof (Example 24).

Anti-inflammatory activity is determined by the Arachidonic Acid-Induced Mouse Ear Edema Assay [Young, et al., *J. Invest. Derm., 82:* 367-371 (1984)] (Example 25).

Anti-inflammatory activity is also determined by the Adjuvant Arthritis assay [Pearson, C.M., *Proc. Soc. Exp. Biol. Med., 91*:95-101 (1956)], or modifications thereof (Example 26).

Anti-autoimmune activity in treating autoimmune disease can be determined utilizing the survivability of MRL/lpr mice described by Theofilopoulos, et al., *Advances in Immunology,* Vol 37, pages 269-390 (1985) on pages 274-276, or a modification thereof (Example 27).

Analgetic activity is determined by the Phenylquinone-induced Mouse Writhing Assay [Hendershot, et al., *J. Pharmacol. Exp. Ther., 125:* 237-240 (1959)] (Example 28).

### ADMINISTRATION

The compounds of this invention are administered at a therapeutically effective dosage, i.e., that amount which, when administered to a mammal in need thereof, is sufficient to effect treatment, as described above (for example, to reduce or otherwise treat inflammation, pain and/or pyrexia in the mammal). Administration of the active compounds and salts described herein can be via any of the accepted modes of administration for agents that serve similar utilities.

The level of the drug in a formulation can vary within the full range employed by those skilled in the art, e.g., from about 0.01 percent weight (%w) to about 99.99%w of the drug based on the total formulation and about .01%w to 99.99%w excipient. Preferably the drug is present at a level of about 10%w to about 70%w.

Generally, an acceptable daily dose is of about 0.001 to 50 mg per kilogram body weight of the recipient per day, preferably about 0.05 to 25 mg per kilogram body weight per day, and most preferably about 0.01 to 10 mg per kilogram body weight per day. Thus, for administration to a 70 kg person, the dosage range would be about 0.07 mg to 3.5 g per day, preferably about 3.5mg to 1.75 g per day, and most preferably about 0.7 mg to 0.7 g per day depending upon the individuals and disease state being treated. Such use optimization is well within the ambit of those of ordinary skill in the art.

Administration can be via any accepted systemic or local route, for example, via parenteral, oral (particularly for infant formulations), intravenous, nasal, bronchial inhalation (i.e., aerosol formulation), transdermal or topical routes, in the form of solid, semi-solid or liquid dosage forms, such as for example, tablets, suppositories, pills, capsules, powders, solutions, suspensions, aerosols, emulsions or the like, preferably in unit dosage forms suitable for simple administration of precise dosages. The compositions will include a conventional pharmaceutical carrier or excipient and an active compound of Formula I and, in addition, may include other medicinal agents, pharmaceutical agents, carriers, adjuvants, etc. Carriers can be selected from the various oils, including those of petroleum, animal, vegetable or synthetic origin, for example, peanut oil, soybean oil, mineral oil, sesame oil, and the like. Water, saline, aqueous dextrose, and glycols are preferred liquid carriers, particularly for injectable solutions. Suitable pharmaceutical carriers include starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water, ethanol, and the like. Other suitable pharmaceutical carriers and their formulations are described in *"Remington's Pharmaceutical Sciences"* by E. W. Martin.

If desired, the pharmaceutical composition to be administered may also contain minor amounts of non-toxic auxiliary substances such as wetting or emulsifying agents, pH buffering agents and the like, such as for example, sodium acetate, sorbitan monolaurate, triethanolamine oleate, etc.

The compounds of this invention are generally administered as a pharmaceutical composition which comprises a pharmaceutical excipient in combination with a compound of formula I wherein R¹ and R² are defined as above and additionally wherein R¹ can be hydrogen or formyl. The level of the drug in a formulation can vary within the full range employed by those skilled in the art, e.g., from about .01 percent weight (%w) to about 99.99%w of the drug based on the total formulation and about .01%w to 99.99%w excipient. Preferably, the formulation will be about 3.5 to 60% by weight of the pharmaceutically active compound, with the rest being suitable pharmaceutical excipients.

### INTRAVENOUS ADMINISTRATION

Intravenous injection has proven to be an important route of administration for therapeutic agents. The compounds of the present invention can be administered via this route, for example, by dissolving the compound, ester, ether, N-oxide or salt in a suitable solvent (such as water or saline) or incorporation in a liposomal formulation followed, by dispersal into an acceptable infusion fluid. A typical daily dose of a compound of the invention can be administered by one infusion, or by a series of infusions spaced over periodic intervals.

### ORAL ADMINISTRATION

Oral administration can be used to deliver the compound of formula I wherein R¹ and R² are defined as above and additionally wherein R¹ can be hydrogen or formyl using a convenient daily dosage regimen which can be adjusted according to the degree of affliction or for renal impairment, or to compensate for the toxic effects of other medications administered contemporaneously. For such oral administration, a pharmaceutically acceptable, non-toxic composition is formed by the incorporation of any of the normally employed excipients, such as, for example, pharmaceutical grades of mannitol, lactose, starch, magnesium stearate, sodium saccharine, talcum, cellulose, glucose, gelatin, sucrose, magnesium carbonate, and the like. Such compositions take the form of solutions, suspensions, tablets, pills, capsules, powders, sustained release formulations and the like. Such compositions may contain between .01 wt/wt% and 99.99 wt/wt% of the compound of formula I wherein R¹ and R² are defined as above and additionally wherein R¹ can be hydrogen of formyl but preferably such compositions will contain between 25 wt/wt% and about 80 wt/wt%.

Preferably the compositions will take the form of a capsule, pill or tablet and thus the composition will contain, along with the active ingredient, a diluent such as lactose, sucrose, dicalcium phosphate, and the like; a disintegrant such as starch or derivatives thereof; a lubricant such as magnesium stearate and the like; and a binder such as a starch, polyvinylpyrrolidone, gum acacia, gelatin, cellulose and derivatives thereof, and the like. For oral administration to infants, a liquid formulation (such as a syrup or suspension) is preferred.

### AEROSOL ADMINISTRATION

Aerosol administration is an effective means for delivering a therapeutic agent directly to the respiratory tract. Some of the advantages of this method are: 1) it circumvents the effects of enzymatic degradation, poor absorption from the gastrointestinal tract, or loss of the therapeutic agent to the hepatic first-pass effect; 2) it administers therapeutic agents which would otherwise fail to reach their target sites in the respiratory tract due to their molecular size, charge or affinity to extra-pulmonary sites; 3) it provides for fast absorption into the body via the alveoli of the lungs; and 4) it avoids exposing other organ systems to the therapeutic agent, which is important where exposure might cause undesirable side effects. For these reasons, aerosol administration is particularly advantageous for treatment of asthma, local infections of the lung, and other diseases or disease conditions of the lung and respiratory tract.

There are three types of pharmaceutical inhalation devices, nebulizers inhalers, metered-dose inhalers (MDI) and dry powder inhalers (DPI). Nebulizer devices produce a stream of high velocity air that causes the therapeutic agent (which has been formulated in a liquid form) to spray as a mist which is carried into the patient's respiratory tract. MDIs typically have the formulation packaged with a compressed gas. Upon actuation, the device discharges a measured amount of therapeutic agent by compressed gas, thus affording a reliable method of administering a set amount of agent.

DPIs administer therapeutic agents in the form of a free flowing powder that can be dispersed in the patient's inspiratory air-stream during breathing by the device. In order to achieve a free flowing powder, the therapeutic agent is formulated with an excipient, such as lactose. A measured amount of the therapeutic agent is stored in a capsule form and is dispensed with each actuation. Examples of DPIs being used are Spinhaler° (for the administration of disodium cromoglycate), Rotahaler° (for albuterol) and Turbuhaler° (for terbutaline sulfate). All of the above methods can be used for administering the present invention, particularly for the treatment of asthma and other similar or related respiratory tract disorders.

### LIPOSOMAL FORMULATIONS

Pharmaceutical formulations based on liposomes have recently reached human clinical trials. Their benefits are believed related to favorable changes in tissue distribution and pharmacokinetic parameters that result from liposome entrapment of drugs, and may be applied to the compounds of the present invention by those skilled in the art.

The formulations can be designed to either target drug to disease sites [see: Lopez-Berestein et al., *J. Infect. Dis., 151:* 704-710 (1985); Gotfredsen et al., *Biochemical Pharmacology,* 32: 3389-3396 (1983)]; or to the reticuloendothelial system [see Eppstein et al., *Int. J. Immunotherapy, 2:* 115-126 (1986)], to increase duration of drug action [see: Gabizon et al., *Cancer Res.,* 42: 4734 (1982); Eppstein et al., *Delivery Systems for Peptide Drugs,* Eds. S.S. Davis, L. Illum and E. Tomlinson, Plenum Pub. Corp., New York, pp. 277-283; C.A. Hunt, *Biochemica et Biophysica Acta., 719*: 450-463 (1982); and Senior et al., *Biochemica et Biophysica Acta., 839:* 1-8 (1985)], or to divert a drug away from organs that are particularly sensitive to its toxic effects [see: Weinstein et al., *Pharmac. Ther.,* 24: 207-233 (1983); Olson et al., *Eur. J. Cancer Clin. Oncol., 18:* 167-176 (1982); and Gabzion et al., *supra.*]

Controlled release liposomal liquid pharmaceutical formulations for injection or oral administration are described in U.S. Patent No. 4,016,100. Liposomal applications for oral drug delivery of a lyophilized liposome/peptide drug mixture filled into intestine capsules have also been suggested, see U.S. Patent No. 4,348,384.

### SUPPOSITORIES

For systemic administration via suppository, traditional binders and carriers include, for example, polyalkaline glycol or triglycerides [e.g., PEG 1000 (96%) and PEG 4000 (4%)] . Such suppositories may be formed from mixtures containing active ingredients in the range of from about 0.5 wt/wt% to about 10 wt/wt%; preferably from about 1 wt/wt% to about 2 wt/wt%.

### LIQUIDS

Liquid pharmaceutically administrable compositions can, for example, be prepared by dissolving, dispersing, etc. an active compound (about 0.5% to about 20%), as described above, and optional pharmaceutical adjuvants in a carrier, such as, for example, water, saline, aqueous dextrose, glycerol, ethanol and the like, to thereby form a solution or suspension.

Actual methods of preparing such dosage forms are known, or will be apparent, to those skilled in this art; for example, see *Remington's Pharmaceutical Sciences,* Mack Publishing Company, Easton, Pennsylvania, 16th Ed., 1980. The composition to be administered will, in any event, contain a quantity of the active compound(s) in a pharmaceutically effective amount for relief of the particular condition being treated in accordance with the teachings of this invention.

### EXAMPLES

The following examples are given to enable those skilled in the art to more clearly understand and to practice the present invention. They should not be considered as limiting the scope of the invention, but merely as being illustrative and representative thereof.

### EXAMPLE 1

### PREPARATION OF 4-(4-AMINOBENZYL)PYRIDINE

### 1A. Formula 2, Where R¹ is 4-Pyridylmethyl

4-(4-Nitrobenzyl)pyridine (214.22 mg) was combined with absolute ethanol (10 mL) and Sncl₂.2H₂O (225.63 mg). The reaction mixture was heated to 70°C under nitrogen for 2 hours. The progress of the reaction was monitored by thin-layer chromatography (e.g., an aliquot of the mixture was collected, neutralized with saturated NaHCO₃ to pH 7-8, extracted into ethyl acetate). When all of the starting material had been converted, the reaction mixture was allowed to cool to room temperature, neutralized with saturated NaHCO₃ to pH 7-8 and extracted with ethyl acetate. The organic layers were collected and dried over MgSO₄. The solvents were removed and the residue was triturated with ethyl ether. The desired product, i.e., 4-(4-aminobenzyl)pyridine, was filtered out of the solution as cream colored crystals and air dried, mp 159.1°C - 160.3°C; elemental analysis:
[talc(found)] C: 78.2 (78.12), H: 6.56 (6.45), and N: 15.20 (15.33).

### 1B. Compounds of Formula 2, Where R¹ Is Varied

Other desired compounds of Formula 2 can be prepared by following the procedures described in Example 1A, and using different starting compounds. For example: 4-isopropylnitrobenzene, 4-methylnitrobenzene, 4-benzylnitrobenzene, 4-ethylnitrobenzene, 4-(3-propenyl)nitrobenzene, 4-propylnitrobenzene, 4-butylnitrobenzene, 4-pentylnitrobenzene, 4-hexylnitrobenzene, 4-methoxynitrobenzene, 4-ethoxynitrobenzene, 4-(trifluoromethyl)nitrobenzene, 4-(3-pyridylmethyl)nitrobenze, 4-(2-pyridylmethyl)nitrobenzene, 4-(cyclopentylmethyl)nitrobenzene, 4-(cyclopropylmethyl)nitrobenzene, 4-(thiomethyl)nitrobenzene, and 4-(methylsulfonylmethyl)nitrobenzene can be converted to the corresponding (substituted) anilines.

### EXAMPLE 2

### PREPARATION OF 4-(4-AMINO-3-BROMOBENZYL)PYRIDINE

### 2A. Formula 3, Where R¹ is 4-Pyridylmethyl

A solution of N-bromosuccinimide (1.4 gm) in 10 mL dimethylformamide was added in a dropwise manner to a solution of 4-(4-aminobenzyl)pyridine (1.5 gm) in 10 mL of dimethylformamide. The reaction flask was wrapped in aluminum foil to prevent exposure of the reagents to light. The reaction mixture was stirred at room temperature for 3 hours. The progress of the reaction was monitored by thin-layer chromatography. When the reaction was completed, the reaction mixture was combined with 100 mL of H₂O with stirring.
A reddish brown precipitate was formed, filtered out of solution, washed with H₂O, and air dried. The solid was dissolved in ethyl acetate, back washed with saturated NaCl, dried over MgSO₄ and triturated with ethyl ether. The solid was filtered from the solution and air dried yielding 1.6 gm of 4-(4-amino-3-bromobenzyl)pyridine as reddish/tan crystals. mp 102.7°C-103.9°C;

### 2B. Other Prepared Compounds of Formula 3, Where R¹ Is Varied

Other compounds of Formula 3 were also prepared following the procedures described in Example 2A, and substituting 4-(4-aminobenzyl)-pyridine with different starting compounds. For example, following is a list of starting compounds and the corresponding desired compounds that were obtained by following the above-referenced procedure:
4-isopropylaniline was converted to 2-bromo-4-isopropylaniline,
4-methylaniline was converted to 2-bromo-4-methylaniline,
4-benzylaniline was converted to 4-benzyl-2-bromoaniline, and
4-(4-pyridylmethyl)aniline was converted to 2-bromo-4-(4-pyridylmethyl)aniline.

### 2C. Compounds of Formula 3, Where R¹ Is Varied

In addition, other desired compounds of Formula 3 can be prepared by following the procedures described in Example 2A, and using different starting compounds.

For example, 4-ethylaniline, 4-(3-propenyl)aniline, 4-propylaniline, 4-butylaniline, 4-pentylaniline, 4-hexylaniline, 4-trifluoromethylaniline, 4-(3-pyridylmethyl)aniline, 4-(2-pyridylmethyl)aniline,4-(cyclopentylmethyl)aniline, 4-(cyclopropylmethyl)aniline,4-(methylthiomethyl)aniline,and 4-(methylsulfonylmethyl)aniline can be converted to the corresponding 2-bromo-4-(substituted)-anilines.

### EXAMPLE 3

### PREPARATION OF 3-CHLOROBENZENE BORONIC ACID

### 3A. Formula 3B Where R² is 3-chlorophenyl

A solution of trimethylborate in 200 mL of ethyl acetate was cooled to -65°C. 3-Chlorobenzene magnesium chloride (0.8 M, 60 mL), i.e., a Grignard reagent, was added to the solution in a dropwise manner over 20 minutes. The mixture was kept in the temperature range of -60°C to -70°C and stirred. After 30 minutes, the mixture was allowed to warm to 0°C and stirred for 1 hour. The mixture was quenched with H₂O (25 mL) and stirred at room temperature for 1 hour. The solvent was removed and the remaining mass was extracted with ethyl ether (3 x 100 mL). The organic layers were combined and washed with H₂O (2 x 50 mL), dilute HCl (2 x 100 mL), H₂O (2 x 50 mL) and brine (1 x 50 mL). The organic layer was dried over MgSO₄, concentrated and allowed to stand. 100 mL of hexanes was added and the solution was stirred for 1 hour. The solution was filtered and allowed to air dry yielding 4.6 g of 3-chlorobenzene boronic acid as a white solid.

### 3B. Compounds of Formula 3B where R² is optionally substituted

Other desired compounds of Formula 3B can be prepared by following the procedures described in Example 3A, and using different starting compounds. For example, 3-chloro-4-fluorobenzene magnesium chloride, 4-chlorobenzene magnesium chloride, benzene magnesium bromide, 3,4-dichlorobenzene magnesium bromide, 3-bromobenzene magnesium bromide, and 3-(trifluoromethyl)benzene magnesium bromide can be converted to the corresponding (substituted) benzene boronic acids.

### EXAMPLE 4

### PREPARATION OF 4-[4-AMINO-3-(3-NITROPHENYL)-BENZYL]PYRIDINE

### 4A. Formula 4, Where R¹ is 4-Pyridylmethyl, R² is 3-Nitrophenyl.

4-(4-Amino-3-bromobenzyl)pyridine (1.0 gm), 3-nitrobenzene boronic acid (0.63 gm), palladium tetrakis triphenylphosphine (0.47 gm), methanol (6.5 mL), 2.0 M Na₂CO₃ (1.9 mL) and benzene (32 mL) were combined in a reaction flask that was wrapped in aluminum foil (to prevent exposure of the reagents to light). The reaction mixture was heated under reflux for 6 hours. The progress of the reaction was monitored by thin-layer chromatography (9:1 hexane:ethyl acetate). When the starting material was converted, the reaction mixture was allowed to cool and the solvents were removed. Ethyl acetate was added to the residue, the resultant solution was filtered through a pad of Na₂SO₄, and concentrated. The product was isolated by preparative thin-layer chromatography (9:1 hexane:ethyl acetate) yielding 983 mg of 4-[4-amino-3-(3-nitrophenyl)benzyl]pyridine as an orange oil.

Characteristic analytical data: ms m/e 305(M+); ¹H NMR (CDCl₃) δ 3.74 (bs, 2H), 3.92 (s, 2H), 6.77 (d, 1H, J=8.1 Hz), 6.95 (d, 1H, J=2.1 Hz), 7.03 (dd, 1H, J=2.1 Hz, J=8.1 Hz), 7.14 (d, 1H, J=5.7 Hz), 7.62 (dd, 1H, J=8.2 Hz, J=7.7 Hz), 7.82 (ddd, 1H, J=7.7 Hz, J=1.9 Hz, J=2.5Hz), 8.21 (ddd, 1H, J=8.2 Hz, J=2.5 Hz, J=1.9Hz), 8.34 (dd, 1H, J=1.9 Hz, J=1.9 Hz), and 8.5 (dd, 1H, J=5.7 Hz).

### 4B. Other Compounds of Formula 4, Where R¹ and R² Are Varied.

Other compounds of Formula 4 were also prepared following the procedures described in Example 4A, and substituting for 4-(4-amino-3-bromobenzyl)pyridine and 3-nitrobenzene boronic acid with different starting compounds. For example, following is a list of starting compounds and the corresponding desired compounds that were obtained by following the above-referenced procedure:
2-bromo-4-isopropylaniline and benzene boronic acid were combined to form 2-phenyl-4-isopropylaniline,
2-bromo-4-isopropylaniline and 3-nitrobenzene boronic acid were combined to form 2-(3-nitrophenyl)-4-isopropylaniline,
4-benzyl-2-bromo-aniline and 3-nitrobenzene boronic acid were combined to form 4-benzyl-2-(3-nitrophenyl)-aniline,
2-bromo-4-methylaniline and 3-chloro-4-fluorobenzene boronic acid were combined to form 2-(3-chloro-4-fluorophenyl)-4-methylaniline,
2-bromo-4-isopropylaniline and 3-chloro-4-fluorobenzene boronic acid were combined to form 2-(3-chloro-4-fluorophenyl)-4-isopropylaniline,
4-benzyl-2-bromo-aniline and 3-chloro-4-fluorobenzene boronic acid were combined to form 4-benzyl-2-(3-chloro-4-fluorophenyl)-aniline,
2-bromo-4-(4-pyridylmethyl)aniline and 3-chloro-4-fluorobenzene boronic acid were combined to form 2-(3-chloro-4-fluorophenyl)-4-(4-pyridylmethyl)aniline,
2-bromo-4-methylaniline and 4-chlorobenzene boronic acid were combined to form 2-(4-chlorophenyl)-4-methylaniline,
2-bromo-4-isopropylaniline and 4-chlorobenzene boronic acid were combined to form 2-(4-chlorophenyl)-4-isopropyl aniline, and 2-bromo-4-benzylaniline and 4-chlorobenzene boronic acid were combined to form 2-(4-chlorophenyl)-4-benzyl- aniline.

### 4C. Compounds of Formula 4, Where R¹ and R² Are Varied

In addition, other desired compounds of Formula 4 can be prepared by following the procedures described in Example 4A, and using different starting compounds. For example,
2-bromo-4-ethylaniline and 3-(trifluoromethyl)benzene boronic acid can be combined to form 2-(3-trifluoromethylphenyl)-4-ethylaniline,
2-bromo-4-isopropylaniline and 3,4-dichlorobenzene boronic acid can be combined to form 2-(3,4-dichlorophenyl)-4-isopropylaniline,
2-bromo-4-(3-propenyl)aniline and 3-nitrobenzene boronic acid can be combined to form 2-(3-nitrophenyl)-3-propenylaniline,
2-bromo-4-propylaniline and 3-nitrobenzene boronic acid can be combined to form 2-(3-nitrophenyl)-4-propylaniline,
2-bromo-4-(*n*-butyl)aniline and 3-nitrobenzene boronic acid can be combined to form 2-(3-nitrophenyl)-4-(*n*-butyl)aniline,
2-bromo-4-(*n*-hexyl)aniline and benzene boronic acid can be combined to form 2-phenyl-4-(*n*-hexyl)aniline,
2-bromo-4-pentylaniline and 3-nitrobenzene boronic acid can be combined to form 2-(3-nitrophenyl)-4-pentylaniline,
2-bromo-4-benzylaniline and 3,4-dichlorobenzene boronic acid can be combined to form 2-(3,4-dichlorophenyl)-4-benzylaniline,
2-bromo-4-cyclopentylmethylaniline and 3-chlorobenzene boronic acid can be combined to form 2-(3-chlorophenyl)-4-cyclopentylmethylaniline,
2-bromo-4-cyclopropylmethylaniline and 3-nitrobenzene boronic acid can be combined to form 2-bromo-4-cyclopropyl- methylaniline,
2-bromo-4-(methylthiomethyl)aniline and 3-chlorobenzene boronic acid can be combined to form 2-(3-chlorophenyl)-4-(methylthiomethyl)aniline,
2-bromo-4-(methylsulfonylmethyl)aniline and benzene boronic acid can be combined to form 2-phenyl-4-(methylsulfonyl- methyl)aniline,
2-bromo-4-(cyclopentylmethyl)aniline and 2,3-dichlorobenzene boronic acid can be combined to form 2-(2,3-dichlorophenyl)-4-(cyclopentylmethyl)aniline, and
2-bromo-4-(cyclopentylmethyl)aniline and 3-nitrobenzene boronic acid can be combined to form 2-(3-nitrophenyl)-4-(cyclopentylmethyl)aniline.

### EXAMPLE 5

### PREPARATION OF 6-(4-PYRIDYLMETHYL)-8-(3-NITROPHENYL)QUINOLINE

### 5A. Formula I, Where R¹ is 4-Pyridylmethyl, R² is 3-Nitrophenyl.

4-[4-Amino-3-(3-nitrophenyl)benzyl]pyridine(600 mg) was combined with glycerol (489 mg) and arsenic pentoxide (325 mg) under nitrogen. The reaction mixture was heated to 100°C for 30 minutes. Concentrated sulfuric acid (406 mg) was added in a dropwise manner and the reaction mixture was further heated to 150°C for 2 hours. The reaction was monitored by TLC (9:1, hexane:ethyl acetate). When TLC indicated that greater than 90% of the starting material had been converted, the reaction mixture was removed from the heat, ice (approximately 1 gm) was added, and NH₄OH was added to basify the mixture. A precipitate was filtered out of the mixture, washed with H₂O and air dried.
The resulting solid was suspended in hot ethyl acetate and filtered. The filtrate was concentrated and chromatographed by preparative thin-layer chromatography (4:1, hexane:ethyl acetate), the band with the higher R_{f} value was isolated yielding 181 mg of 6-(4-pyridylmethyl)-8-(3-nitrophenyl)quinoline as a viscous yellow oil. The product was recrystallized from diethyl ether as a light yellow solid.

Characteristic analytical data: mp 131°C-143°C; elemental analysis, talc(found) C: 73.89 (73.90), H: 4.43 (4.50), and N: 12.31 (12.30); ¹H NMR CDCl₃ 4.21 (s, 2H), 7.21 (d, 2H, J=5.9 Hz), 7.48 (dd, 1H, J=8.3 Hz, J=4.2 Hz), 7.6 (d, 1H, J=2 Hz), 7.66 (dd, 1H, J=8 Hz, J=7.7 Hz), 7.7 (d, 1H, J=2Hz), 8.03 (ddd, 1H, J=7.7 Hz, J=1.4 Hz, J=1.2 Hz), 8.2 (dd, 1H, J=8.3 Hz, J=1.7 Hz, J=1.2 Hz), 8.28 (ddd, 1H J=8 Hz, J=1.44 Hz, J=1.2 Hz), 8.55(m, 3H), 8.93 (dd, 1H, J=4.2 Hz, J=1.7 Hz).

### 5B. Other Compounds of Formula I, Where R¹ and R² Are Varied.

Other compounds of Formula I were also prepared following the procedures described in Example 5A, and substituting 4-[4-amino-3-(3-nitrophenyl)benzyl]pyridine with different starting compounds. For example, following is a list of starting compounds and the corresponding desired compounds that were obtained by following the above-referenced procedure:
2-phenyl-4-isopropylaniline was converted to 6-isopropyl-8-phenylquinoline as an oil,
2-(3-nitrophenyl)-4-isopropylaniline was converted to 6-isopropyl-8-(3-nitrophenyl)quinoline as an oil,
4-benzyl-2-(3-nitrophenyl)aniline was converted to 6-benzyl-8-(3-nitrophenyl)quinoline, mp 101°C-103°C,
2-(3-chloro-4-fluorophenyl)-4-methylaniline was converted to 6-methyl-8-(3-chloro-4-fluorophenyl)quinoline, mp 96°C-114°C,
2-(3-chloro-4-fluoro)-4-isopropylaniline was converted to 6-isopropyl-8-(3-chloro-4-fluorophenyl) quinoline as an oil,
4-benzyl-2-(3-chloro-4-fluorophenyl)aniline was converted to 6-benzyl-8-(3-chloro-4-fluorophenyl)quinoline as an oil,
2-(3-chloro-4-fluorophenyl)-4-(4-pyridylmethyl)aniline was converted to 6-(4-pyridylmethyl)-8-(3-chloro-4-fluorophenyl)quinoline as an oil,
2-(4-chlorophenyl)-4-methylaniline was converted to 6-methyl-8-(4-chlorophenyl)quinoline,mp 96°C-98°C,
2-(4-chlorophenyl)-4-isopropylaniline was converted to 6-isopropyl-8-(4-chlorophenyl)quinoline as an oil, and
4-benzyl-2-(4-chlorophenyl)aniline was converted to 6-benzyl-8-(4-chlorophenyl)quinoline as an oil.

### 5C. Formula I, Where R¹ and R² Are Varied

In addition, other desired compounds of Formula I can be prepared by following the procedures described in Example 5A, and using different starting compounds. For example,
2-(3-trifluoromethylphenyl)-4-ethylaniline can be converted to 6-ethyl-8-(3-trifluoromethylphenyl)quinoline,
2-(3,4-dichlorophenyl)-4-isopropylaniline can be converted to 6-isopropyl-8-(3,4-dichlorophenyl)quinoline,
2-(3-nitrophenyl)-4-(3-propenyl)aniline can be converted to 6-(3-propenyl)-8-(3-nitrophenyl)quinoline,
2-(3-nitrophenyl)-4-propylaniline can be converted to 6-propyl-8-(3-nitrophenyl)quinoline,
2-(3-nitrophenyl)-4-butylaniline can be converted to 6-butyl-8-(3-nitrophenyl)quinoline,
2-(phenyl)-4-hexylaniline can be converted to 6-hexyl-8-(phenyl)quinoline,
2-(3-nitrophenyl)-4-pentylaniline can be converted to 6-pentyl-8-(3-nitrophenyl)quinoline,
2-(3,4-dichlorophenyl)-4-benzylaniline can be converted to 6-benzyl-8-(3,4-dichlorophenyl)quinoline,
2-(3-methoxycarbonylphenyl)-4-(3-pyridylmethyl)anilinecan be converted to 6-(3-pyridylmethyl)-8-(3-methoxycarbonyl-quinoline,
2-(3-methoxycarbonylphenyl)-4-(2-pyridylmethyl)anilinecan be converted to 6-(2-pyridylmethyl)-8-(3-methoxycarbonyl-quinoline,
2-(3-chlorophenyl)-6-cyclopentylmethylaniline can be converted to 6-cyclopentylmethyl-8-(3-chlorophenyl)quinoline,
2-(3-nitrophenyl)-4-cyclopropylmethylaniline can be converted to 6-cyclopropylmethyl-8-(3-nitrophenyl)quinoline,
2-(3-chlorophenyl)-4-(methylthiomethyl)aniline can be converted to 6-methylthiomethyl-8-(3-chlorophenyl)quinoline,
2-phenyl-4-(methylsulfonylmethyl)aniline can be converted to 6-methylsulfonylmethyl-8-(phenyl)quinoline,
2-(3,4-dichlorophenyl)-4-(cyclopentylmethyl)aniline can be converted to 6-cyclopentylmethyl-8-(3,4-dichlorophenyl)quinoline, and
2-(3-nitrophenyl)-4-(cyclohexylmethyl)aniline can be converted to 6-cyclohexylmethyl-8-(3-nitrophenyl)quinoline.

### EXAMPLE 6

### PREPARATION OF 6-ISOPROPYL-8-BROMOQUINOLINE

### 6A. Formula 5 Where R¹ Is Isopropyl

2-Bromo-4-isopropylaniline (3.0 gm) [obtained from Aldrich Chemical Co.], glycerol (2.8 Ml) and arsenic pentoxide (3.22 g) were combined and heated to 100°C. H₂SO₄ (concentrated, 1.9 Ml) was added to the reaction mixture in a dropwise manner. The mixture was then heated to 150°C for 2.5 hours. The resulting black oil was added in a dropwise manner to a stirring mixture of saturated NaHCO₃ (300 Ml) and ethyl acetate (100 Ml). After completion of the addition, the reaction mixture was stirred for 30 minutes. The reaction mixture was then extracted with ethyl acetate (2 x 200 Ml). The organic layers were washed with brine, dried over MgSO₄, filtered and concentrated. The resulting material was further purified and isolated by chromatography on silica gel (30:70 ethyl acetate/hexanes) which yielded 2.5 g of 6-isopropyl-8-bromoquinoline as a brown oil.

Characteristic analytical data: elemental analysis calc.(found) : C 57.62 (57.65), H 4.84 (4.78), and N 5.60 (5.57); ¹H NMR (CDCl₃) δ 1.34 (d, 6H, J=6.9 Hz), 3.05 (m, 1H, J=6.9 Hz), 7.42 ((dd, 1H, J=4.2 Hz, J=8.3 Hz), 7.57 (d, 1H, J=1.8 Hz), 7.97 (d, 1H, J=1.8 Hz), 8.1 (dd, 1H, J=8.3 Hz, J=1.7 Hz), and 8.9 (dd, 1H, J=4.2 Hz, J=1.7 Hz).

### EXAMPLE 7

### PREPARATION OF 6-ISOPROPYL-8-(3-NITROPHENYL)QUINOLINE HYDROCHLORIDE

### 7A. Formula I Where R¹ Is Isopropyl and R² Is 3-Nitrophenyl.

6-Isopropyl-8-bromoquinoline (1.15 g) was dissolved in 46 Ml of ethanol/benzene (1:1). 3-Nitrobenzene boronic acid (1.4 g), Na₂CO₃ (2M, 9.2 Ml) and tetrakis(triphenylphosphine)palladium (0.23 g) were added successively. The reaction mixture was refluxed for 6 hours and then cooled and concentrated. The resulting residue was partitioned between 75 mL H₂O and 100 mL of ethyl acetate, and extracted with ethyl acetate (2 x 100 mL). The extracts were then dried over MgSO₄, filtered, concentrated and chromatographed on silica gel (20:80 ethyl acetate:hexane) yielding 1.2 g of 6-isopropyl-8-(3-nitrophenyl)- quinoline as a slightly impure yellow oil.

The desired product was obtained in its pure form by recrystallization of its hydrochloride salt. The oil was dissolved in 25 mL of 10% methanol:methylene chloride, to which a solution of 1M HCl/Et₂O (100 mL) was added. The solution was stirred for 10 minutes and then concentrated. The resulting white solid was recrystallized from ethyl acetate:ethanol yielding 0.74 g of 6-isopropyl-8-(3-nitrophenyl)quinoline as pale yellow crystals.

Characteristic analytical data: elem. anal. talc. (found): C 65.75 (65.92), H 5.21 (5.21), and N 8.52 (8.67); and ¹H NMR (DMSO) δ 1.37 (d, 6H, J=6.9 Hz), 3.2 (m, 1H, J=6.9 Hz), 7.81 (m, 2H), 7.92 (d, 1H, J=1.9 Hz), 8.09 (d, 1H, J=1.9 Hz), 8.11 (m, 1H), 8.43 (m, 1H), 8.53 (m, 1H), 8.7 (dd, 1H, J=1.5 Hz, J=8.3 Hz), 8.9 (dd, 1H, J=4.7 Hz, J=8.3 Hz).

### EXAMPLE 8

### PREPARATION OF 6-METHYL-8-BROMOQUINOLINE

### 8A. Formula 5 Where R¹ is Methyl and X is Bromo

A slurry of 2-bromo-4-methylaniline (5 g), glycerol (6.7 g), and arsenic pentoxide (3.9 g) was formed and heated to 100°C for 30 minutes. Concentrated H₂SO₄ (4.9 g) was added dropwise and the mixture was heated to 150°C for two hours. The progress of the reaction was monitored by thin-layer chromatography (9:1 hexane:ethyl acetate). Upon completion, the reaction mixture was worked up by adding water, basifying with saturated NaHCO₃, extracting with ethyl acetate, and drying the organic layer over MgSO₄. The desired product was isolated and purified by column chromatography (9:1 hexane:ethyl acetate), yielding 6-methyl-8-bromoquinoline (1.8 g) as a yellow liquid.

Characteristic analytical data: ¹H NMR (CDCl₃) δ 2.5 (s, 3H), 7.4 (dd, 1H, J=8.3 Hz, J=4.3 Hz), 7.52 (bs, 1H), 7.9 (d, 1H, J=1.8 Hz), 8.07 (dd, 1H, J=8.3 Hz, J=1.7 Hz), 8.88 (dd, 1H, J=4.3 Hz, J=1.7 Hz).

### 8B. Formula 5 Where R¹ Is Varied

Following the procedures described in Example 8A the following desired compounds of Formula 5 can be obtained from the indicated starting compounds. For example,
2-bromo-4-isopropylaniline and glycerol can be combined to form 6-isopropyl-8-bromoquinoline,
2-bromo-4-methylaniline and glycerol can be combined to form 6-methyl-8-bromoquinoline,
2-bromo-4-benzylaniline and glycerol can be combined to form 6-benzyl-8-bromoquinoline,
2-bromo-4-(4-pyridylmethyl)aniline and glycerol can be combined to form 6-(4-pyridylmethyl)-8-bromoquinoline,
2-bromo-4-ethylaniline and glycerol can be combined to form 6-ethyl-8-bromoquinoline,
2-bromo-4-(3-propenyl)aniline and glycerol can be combined to form 6-(3-propenyl)-8-bromoquinoline,
2-bromo-4-propylaniline and glycerol can be combined to form 6-propyl-8-bromoquinoline,
2-bromo-4-(*n*-butyl)aniline and glycerol can be combined to form 6-(*n*-butyl)-8-bromoquinoline,
2-bromo-4-pentylaniline and glycerol can be combined to form 6-pentyl-8-bromoquinoline,
2-bromo-4-hexylaniline and glycerol can be combined to form 6-hexyl-8-bromoquinoline,
2-bromo-4-(3-pyridylmethyl)aniline and glycerol can be combined to form 6-(3-pyridylmethyl)-8-bromoquinoline,
2-bromo-4-(2-pyridylmethyl)aniline and glycerol can be combined to form 6-(2-pyridylmethyl)-8-bromoquinoline,
2-bromo-4-(cyclopentylmethyl)aniline and glycerol can be combined to form 6-(cyclopentylmethyl)-8-bromoquinoline,
2-bromo-4-(cyclopropylmethyl)aniline and glycerol can be combined to form 6-(cyclopropylmethyl)-8-bromoquinoline,
2-bromo-4-(methylthiomethyl)aniline and glycerol can be combined to form 6-(methylthiomethyl)-8-bromoquinoline, and
2-bromo-4-(methylsulfonylmethyl)aniline and glycerol can be combined to form 6-(methylsulfonylmethyl)-8-bromoquinoline.

### EXAMPLE 9

### PREPARATION OF 6-METHYL-8-(3-NITROPHENYL)QUINOLINE

### 9A. Formula I, Where R¹ is Methyl and R² is 3-Nitrophenyl.

To a mixture of 6-methyl-8-bromoquinoline (1.0 g), 3-nitrobenzene boronic acid (0.63 g), purchased from Lancaster Chemicals, 2M NaCO₃ (1.9 mL), methanol (6.5 mL) and benzene 32 mL) was added palladium tetrakis triphenylphosphine (0.47 g). The mixture was heated under reflux for 6 hours. The progress of the reaction was monitored by thin-layer chromatography. Upon completion, the reaction mixture was cooled and the solvents removed. Ethyl acetate was added to the residue and the solution was filtered through a pad of Na₂SO₄. The solution was concentrated and the desired product was purified and isolated by preparative thin-layer chromatography yielding 6-methyl-8-(3-nitrophenyl)quinoline (983 mg) as a yellow oil. Elemental analysis [calc.(found)] C: 72.72 (72.63), H: 4.58 (4.32), and N: 10.60 (10.72).

### 9B. Formula I, Where R¹ and R² Are Varied

The compounds of Formula I that were prepared following the procedures described in Examples 1-5 (i.e., following Reaction Scheme A) can also be prepared by following the procedures described in Examples 6, 7, 8 and 9 (i.e., following reaction Scheme B).
For example,
6-isopropyl-8-bromoquinoline combined with benzene boronic acid can be converted to 6-isopropyl-8-phenylquinoline,
6-isopropyl-8-bromoquinoline combined with 3-nitrobenzene boronic acid can be converted to 6-isopropyl-8-(3-nitrophenyl)quinoline,
6-benzyl-8-bromoquinoline combined with 3-nitrobenzene boronic acid can be converted to 6-benzyl-8-(3-nitrophenyl)quinoline,
6-methyl-8-bromoquinoline combined with 3-chloro-4-fluorobenzene boronic acid can be converted to 6-methyl-8-(3-chloro-4-fluorophenyl)quinoline,
6-isopropyl-8-bromoquinoline combined with 3-chloro-4-fluoro- benzene boronic acid can be converted to 6-isopropyl-8-(3-chloro-4-fluorophenyl)quinoline,
6-benzyl-8-bromoquinoline combined with 3-chloro-4-fluorobenzene boronic acid can be converted to 6-benzyl-8-(3-chloro-4-fluorophenyl)quinoline,
6-(4-pyridylmethyl)-8-bromoquinoline combined with 3-chloro-4-fluorobenzene boronic acid can be converted to 6-(4-pyridylmethyl)-8-(3-chloro-4-fluorophenyl)quinoline,
6-methyl-8-bromoquinoline combined with 4-chlorobenzene boronic acid can be converted to 6-methyl-8-(4-chlorophenyl) quinoline,
6-isopropyl-8-bromoquinoline combined with 4-chlorobenzene boronic acid can be converted to 6-isopropyl-8-(4-chlorophenyl)quinoline, and
6-benzyl-8-bromoquinoline combined with 4-chlorobenzene boronic acid can be converted to 6-benzyl-8-(4-chlorophenyl)-quinoline.

### 9C. Formula I, Where R¹ and R² Are Varied

In addition, other desired compounds of Formula I can be prepared by following the procedures described in Example 9A, and using different starting compounds. For example,
6-ethyl-8-bromoquinoline and 3,4 dichlorobenzene boronic acid can be combined to form 6-ethyl-8-(3,4-dichlorophenyl)quinoline,
6-(3-propenyl)-8-bromoquinoline and 3-(trifluoromethyl)benzene boronic acid can be combined to form 6-(3-propenyl)-8-(3-trifluoromethylphenyl)quinoline,
6-propyl-8-bromoquinoline and 3-nitrobenzene boronic acid can be combined to form 6-propyl-8-(3-nitrophenyl)quinoline,
6-(*n*-butyl)-8-bromoquinoline and 3-chlorobenzene boronic acid can be combined to form 6-(*n*-butyl)-8-(3-chlorophenyl)quinoline,
6-pentyl-8-bromoquinoline and 3,4-dichlorobenzene boronic acid can be combined to form 6-pentyl-8-(3,4-dichlorophenyl)quinoline,
6-hexyl-8-bromoquinoline and 3-nitrobenzene boronic acid can be combined to form 6-hexyl-8-(3-nitrophenyl)quinoline,
6-(3-pyridylmethyl)-8-bromoquinoline and 3-nitrobenzene boronic acid can be combined to form 6-(3-pyridylmethyl)-8-(3-nitrophenyl)quinoline,
6-(2-pyridylmethyl)-8-bromoquinoline and 3-chlorobenzene boronic acid can be combined to form 6-(2-pyridylmethyl)-8-(3-chlorophenyl)quinoline,
6-(cyclopentylmethyl)-8-bromoquinoline and 3-chloro-4-fluorobenzene boronic acid can be combined to form 6-(cyclopentylmethyl)-8-(3-chloro-4-fluorophenyl)quinoline,
6-(cyclopropylmethyl)-8-bromoquinoline and benzene boronic acid can be combined to form 6-(cyclopropylmethyl)-8-phenyl- quinoline,
6-(methylthiomethyl)-8-bromoquinoline and benzene boronic acid can be combined to form 6-(methylthiomethyl)-8-phenylquinoline, and
6-(methylsulfonylmethyl)-8-bromoquinoline and 3-nitrobenzene boronic acid can be combined to form 6-(methylsulfonyl- methyl)-8-(3-nitrophenyl)quinoline.

### EXAMPLE 10

### PREPARATION OF 8-(3-CHLOROPHENYL)-6-QUINOLINECARBOXALDEHYDE

### 10A. Formula I Where R¹ is Bromomethyl

6-Methyl-8-(3-chlorophenyl)quinoline (prepared following the procedures described in Example 9, Reaction Scheme B) (1.64 g) was combined with 30 mL of carbon tetrachloride and heated to reflux.
N-Bromosuccinamide (1.34 g) and 2,2'-azobis(2-methylpropionitrile) (0.025 g) was added and the reaction mixture was exposed to light from a 250 watt light bulb for 1 hour. The reaction mixture was then stirred for an additional 2 hours. The progress of the reaction was monitored by thin-layer chromatography (9:1 hexane/ethyl acetate). Upon completion of the reaction, the reaction mixture was cooled to 0°C and poured through a 2cm pad of Na₂SO₄ on 1cm pad on silica gel. The filtrate was concentrated yielding 2.26 g of a yellow oil as a mixture of the monobrominated compound as the major product as well as the dibrominated compound as the minor product. The crude oil (1.84 g) was dissolved in 25 mL of chloroform and added dropwise to a solution of tetra n-butylammonium dichromate (19.4 g) in methylene chloride (30 mL) and refluxed for 4 hours. The reaction mixture was then cooled to room temperature, filtered through silica gel, eluted with ether and concentrated. The residue was chromatographed in hexane:ethyl acetate (70:30) to obtain 8-(3-chlorophenyl)-6-quinolinecarboxaldehyde (0.93 g).

Characteristic analytical data: ¹H NMR (CDCl₃) δ 7.41 (m, 2H) 7.52 (dd, 1H, J=5.2 Hz, J=4.2 Hz), 7.6 (m, 1H), 7.71 (m, 1H), 8.19 (d, 1H, J=1.9 Hz), 8.38 (d, 1H, J=1.9 Hz), 8.4 (dd, 1H, J=5.2 Hz, J=1.8 Hz), 9.6 (dd, 1H, J=4.2 Hz, J=1.8 Hz), 10.22 (s, 1H); and elemental analysis for C₁₆H₁₀ClNO, talc(found) C, 71.78 (71.79); H, 3.77 (3.81); N, 5.23 (5.39).

### 10B. Formula I, Where R¹ Is Bromomethyl and R² Is Varied.

Following the procedures described in Example 10A the following desired compounds of Formula I where R' is bromomethyl can be obtained from the indicated starting compounds. For example,
6-methyl-8-(3-nitrophenyl)quinoline can be converted to 8-(3-nitrophenyl)-6-quinolinecarboxaldehyde,
6-methyl-8-phenylquinoline can be converted to 8-phenyl-6-quinolinecarboxaldehyde,
6-methyl-8-(3-chlorophenyl)quinoline can be converted to 8-(3-chlorophenyl)-6-quinolinecarboxaldehyde,
6-methyl-8-(3-trifluoromethylphenyl)quinoline can be converted to 8-(3-trifluoromethylphenyl)-6-quinolinecarboxaldehyde,
6-methyl-8-(3-chlorophenyl)quinoline can be converted to 8-(3-chlorophenyl)-6-quinolinecarboxaldehyde,
6-methyl-8-(3-chloro-4-fluoro-phenyl)quinoline can be converted to 8-(3-chloro-4-fluorophenyl)-6-quinolinecarboxaldehyde,
6-methyl-8-(3,4-dichlorophenyl)quinoline can be converted to 8-(3,4-dichlorophenyl)-6-quinolinecarboxaldehyde, and
6-methyl-8-(4-chlorophenyl)quinoline can be converted to 8-14-chlorophenyl)-6-quinolinecarboxaldehyde.

### EXAMPLE 11

### PREPARATION OF 6-(1-HYDROXYETHYL)-8-(3-CHLOROPHENYL)QUINOLINE

### 11A. Formula I Where R¹ Is 1-hydroxyethyl

8-(3-Chlorophenyl)-6-quinolinecarboxaldehyde (0.8 g) was dissolved in 20 mL of tetrahydrofuran and cooled to -78°C. Methyllithium (4.5 mL, 1.4M) in diethyl ether was added dropwise to the solution over 5 minutes. The solution was stirred for 20 minutes, poured into 50 mL of saturated ammonium chloride, stirred for 5 minutes and extracted with ethyl acetate (2 x 75 mL). The extracts were combined and dried over MgSO₄, and concentrated. The residue was chromatographed on silica gel (30:70 ethyl acetate:hexanes) to obtain 0.72 g of 6-(1-hydroxyethyl)-8-(3-chlorophenyl) quinoline as a yellow oil.

Characteristic analytical data: ¹H NMR (CDCl₃) δ 1.6 (d, 3H, J=6.5 Hz), 5.1 (q, 1H, J=6.5 Hz), 7.4 (m, 3H), 7.55 (m, 1H), 7.67 (m, 1H), 7.7 (d, 1H, J=1.9 Hz), 7.8 (d, 1H, J=1.9 Hz), 8.18 (dd, 1H, J=8.3 Hz, J=1.8 Hz), 8.9 (dd, 1H, J=4.2 Hz, J=1.8 Hz).

### 11B. Formula I, Where R¹ Is Formyl and R² Is Varied

Following the procedures described in Example 11A the following desired compounds of Formula I where R' is formyl can be obtained from the indicated starting compounds. For example,
8-(3-nitrophenyl)-6-quinolinecarboxaldehyde and methyllithium can be converted to 6-(1-hydroxyethyl)-8-(3-nitrophenyl)-quinoline,
8-phenyl-6-quinolinecarboxaldehyde and *n*-butyl magnesium chloride can be converted to 6-(1-hydroxypentyl)-8-phenylquinoline,
8-(3-chlorophenyl)-6-quinolinecarboxaldehydeand ethyllithium can be converted to 6-(1-hydroxypropyl)-8-(3-chlorophenyl)-quinoline,
8-(3-trifluoromethylphenyl)-6-quinolinecarboxaldehyde and hexyl magnesium chloride can be converted to 6-11-hydroxy-heptyl)-8-(3-trifluoromethylphenyl)quinoline,
8-(3-chlorophenyl)-6-quinolinecarboxaldehyde and methyl magnesium chloride can be converted to 6-(1-hydroxyethyl)-8-(3-chlorophenyl)quinoline,
8-(3-chloro-4-fluorophenyl)-6-quinolinecarboxaldehyde and methyllithium can be converted to 6-(1-hydroxyethyl)-8-(3-chloro-4-fluorophenyl)quinoline,
8-(3,4-dichlorophenyl)-6-quinolinecarboxaldehyde and propyllithium can be converted to 6-(1-hydroxybutyl)-8-(3,4-dichlorophenyl)quinoline, and
8-(4-chlorophenyl)-6-quinolinecarboxaldehyde and hexyllithium can be converted to 6-(1-hydroxyheptyl)-8-(4-chlorophenyl)-quinoline.

### EXAMPLE 12

### PREPARATION OF

### 6-(4-PYRIDYL-N-OXIDE-METHYL)-8-(3-NITROPHENYL)QUINOLINE

To a solution of 6-(4-pyridylmethyl)-8-(3-nitrophenyl)quinoline (100 mg) in methylene chloride (10 mL) under nitrogen at room temperature was added m-chloroperoxybenzoic acid (59 mg). The reaction mixture was stirred at room temperature for 3½ hours. The progress of the reaction was monitored by TLC. Upon completion of the reaction, the reaction mixture was worked up by preparative thin-layer chromatography yielding 6-(4-pyridyl-N-oxide-methyl)-8-(3-nitrophenyl)quinoline (87 mg) as creme colored crystals.

Characteristic analytical data: ¹H (CDCl₃) δ 4.21 (s, 2H), 7.13 (d, 2H, J=5.2 Hz), 7.43 (dd, 1H, J=8.2 Hz, J=4.2 Hz), 7.5 (d, 1H, J=1.9 Hz), 7.6 (dd, 1H, J=8 Hz, J=8 Hz), 7.7 (d, 1H, J=1.9 Hz), 7.95 (ddd, 1H J=7.7 Hz, J=1.4 Hz, J=1.3 Hz), 8.13 (d, 2H, J=5.2 Hz), 8.18 (dd, 1H, J=8.2 Hz, J=1.7 Hz), 8.21 (ddd, 1H, J=8.2 Hz, J=J.14, J=1.3 Hz), 8.55 (dd, 1H, J=1.3 Hz, J=1.3 Hz), 8.92 (dd, 1H, J=4.2 Hz, J=1.7 Hz).

### EXAMPLE 13 (REACTION SCHEMES B-1, B)

### PREPARATION OF 6-BROMOMETHYL-8-BROMOQUINOLINE

### Formula 5, Where R¹ Is Bromomethyl and R² Is Bromo

3.7g (16.66 mmol) of 6-Methyl-8-bromoquinoline was dissolved in 85mL of CCl₄ and 3g of N-bromosuccinamide (16.66 mmol) was added followed by a catalytic amount of azobisisobutyronitrile (AIBN). The reaction mixture was heated at reflux under nitrogen for 1.5 hours and irradiated with a lamp, the cooled and pour into water (100mL). The organic layer was separated and washed with H₂O (100mL) and brine (100mL), dried over MgSO₄, filtered and concentrated to give a brown solid. Crystallization from ethyl acetate/hexane yielded 2.84g (9.44 mmol) of light brown crystals. The filtrate was concentrated and the residue crystallized from the same solvent system. A second crop of light brown crystals (0.77g 2.56 mmol) was obtained. Column chromatography on silica gel of the concentrated filtrate in 20% ethyl acetate/hexanes gave 0.59g (1.96 mmol) of 6-bromomethyl-8-bromoquinoline as a brown solid (4.2g total, 13.95 mmol, 84%), mp 156.2-157.8°.

### PREPARATION OF 6-(N-PYRROLIDINYLMETHYL-8-BROMOQUINOLINE

### Formula 5, Where R¹ Is Pyrrolidinylmethyl and R² Is Bromo

0.62g (2.1 mmol) of 6-bromomethyl-8-bromoquinoline was slurried in 15 mL of THF and pyrrolidine (0.38 mL, 4.6 mmol) was added dropwise under nitrogen. The reaction mixture was stirred for 1.5h, and then poured into half saturated NaHCO₃ (50mL) and 25 mL EtOAc. The organic layer was washed with H₂O (20mL), and brine (25mL), dried over MgSO₄, filtered and concentrated. Flash chromatography of the residue on silica gel using 5% MeOH/CH₂Cl₂ yielded 0.44g (1.51 mmol, 72%) of 6-(N-pyrrolidinylmethyl)-8-bromoquinoline as a dark oil.

### PREPARATION OF 6-(N-PYRROLIDINYLMETHYL-8-(3-NITROPHENYL)QUINOLINE

### Formula 5, Where R¹ Is Pyrrolidinylmethyl and R² Is 3-Nitrophenyl

0.32g (1.1 mmol) of 6-(N-pyrrolidinylmethyl)-8-bromoquinoline was dissolved in 10 mL of 1:1 EtOH/benzene under nitrogen. 3-nitrophenyl boronic acid (0.37g, 2.2 mmol), 2M Na₂CO₃ (2.2 mL, 4.4 mmol), and tetrakis(triphenyl-phosphine)palladium Pd(PPh₃)₄ (0.05g, 0.044 mmol) were added successively and the reaction mixture was refluxed for 4h, then cooled and poured into water (15mL) and extracted with EtOAc (15 mL). The organic layer was washed with water (10 mL) and brine (10mL), dried over MgSO₄, filtered and concentrated. The residue was chromatographed using 10% methanol/ CH₂Cl₂. 0.29g (0.87 mmol) of 6-(N-pyrrolidinylmethyl)-8-(3-nitrophenyl)quinoline was obtained as a yellow oil. The oil was dissolved in 15 ml of EtOAc, and the HCL salt was formed with a saturated solution of HCl/EtOAc. The residue was concentrated and crystallized from ethanol/ethyl acetate/ether/hexane to give 0.29g (0.71 mmol, 65%) of 6-(N-pyrrolidinylmethyl)-8-(3-nitro-phenyl)quinoline dihydrochloride as yellow crystals, mp 223-225°C.

### PREPARATION OF 6-(N-PYRROLIDINONYLMETHYL)-8-BROMOQUINOLINE

### Formula 5, Where R¹ Is Pyrrolidinonylmethyl and R² Is Bromo

8-bromo-6-bromomethylquinoline (1.33 mmol) was dissolved in THF/[1,3-dimethyl-3,4,5,6-tetrahydro-2(1H)pyrimidinone (DMPU) and reacted with a solution of NaH (1.6 mmol) and pyrrolidinone (1.6 mmol) at reflux for 24 h to obtain 8-bromo-6-(N-pyrrolidinonylmethyl) quinoline as a yellow oil that was used directly in the next step.

### PREPARATION OF 6-(N-PYRROLIDINONYLMETHYL-8-(3-NITROPHENYL)QUINOLINE

### Formula I, Where R¹ Is Pyrrolidinonylmethyl and R² Is 3-Nitrophenyl

8-bromo-6-(N-pyrrolidinonylmethyl) quinoline (1.05 mmol) was reacted with 3-nitroboronic acid (2.1 mmol), 2M Na2CO3 (4.2 mmol), and Pd(PPh₃)₄ (0.04 mmol) to obtain 8-(3-nitrophenyl)-6-(N-pyrrolidinonylmethyl) quinoline as white crystals (0.55 mmol, 52%), mp 127.4-128.3°C.

### EXAMPLE 14 (REACTION SCHEME C-3)

### PREPARATION OF 6-PYRROLYLMETHYL-8-(3-NITROPHENYL)QUINOLINE

### Formula I, Where R¹ Is Pyrrolylmethyl and R² Is 3-Nitrophenyl

0.6g (1.75 mmol) of freshly distilled pyrrole was added to a slurry of NaH (0.046g, 1.9 mmol) in 5 mL of dry DMF under nitrogen. The reaction mixture was stirred for 10 min and then 0.6g (1.75 mmol) of 6-bromomethyl-8-(3-nitrophenyl)quinoline, dissolved in 5 mL of DMF, was added to form a dark solution. The solution was stirred at room temperature for 2h, then heated at 80°C for 2h. The reaction mixture was cooled and poured into 150 mL of water, extracted with CH₂Cl₂ (4 x 25 mL). The combined organic layers were washed with water (50 mL), brine (50mL), and dried over MgSO₄. Filtration and concentration provided a solid which was purified by flash chromatography on silica gel using 30% ethyl acetate/hexane.
Yield: 0.3g of a yellow oil.
The oil was dissolved in 15 mL of ethyl acetate and the HCl salt was formed from ethyl acetate/HCl. The solution was concentrated and the resulting oil was crystallized from ethanol/ether . Yield: 0.26g (0.64 mmol, 37%) of 6-pyrrolylmethyl-8-(3-nitrophenyl)quinoline dihydrochloride as yellow crystals, mp 183.2-184.8°C.

### PREPARATION OF 6-(PHENYLAMINOMETHYL)-8-(3-NITROPHENYL)QUINOLINE

### Formula I, Where R¹ Is Phenylaminomethyl and R² Is 3-Nitrophenyl

0.5g (1.46 mmol) of 6-bromomethyl-8-(3-nitrophenyl)quinoline was dissolved in 10mL of ethanol. 0.15g (1.61 mmol) of aniline and 0.3g (2.2 mmol) of K₂CO₃ was added. The reaction mixture was refluxed under nitrogen for 24h, then poured into water (50 mL) and ethyl acetate (50 mL) The organic layer was separated and washed with water (30 mL) and brine (30 mL), dried over MgSO₄, filtered, concentrated and purified by flash chromatography using 30% ethyl acetate/hexane. An impure yellow oil was obtained which was further purified by preparative TLC using the same solvent system to obtain 0.2g of yellow oil. Crystallization from ethyl acetate/hexane gave O.lg (0.28 mmol, 19%) of 6-(phenylaminomethyl)-8-(3-nitrophenyl)quinoline as yellow crystals, mp 149.9-150.5°C.

### PREPARATION OF 6-(1,2,4-TRIAZOLYLMETHYL)-8-(3-NITROPHENYL)QUINOLINE

### Formula I, Where R¹ Is 1,2,4-Triazolylmethyl and R² Is 3-Nitrophenyl

6-Bromomethyl-8-12-nitrophenyl)quinoline (1.75 mmol) was reacted with 1,2,4-triazole (3.5 mmol), K₂CO₃ (4.38 mmol), and tetrabutylammonium iodide (0.18 mmol) in refluxing ethyl acetate to obtain 6-(1,2,4-triazolylmethyl)-8-(3-nitrophenyl)quinoline (0.91 mmol, 57%) as yellow crystals, mp 160.7-161.5°C.

### PREPARATION OF 6-(1,2,4-TRIAZOLYLMETHYL)-8-(3-CHLOROPHENYL)QUINOLINE

### Formula I, Where R¹ Is 1,2,4-Triazolylmethyl and R² Is 3-Chlorophenyl

6-Bromomethyl-8-(3-chlorophenyl)quinoline (1.26 mmol) was reacted with a solution of NaH (1.89 mmol) and 1,2,4-triazole (1.89 mmol), in THF for 24h to give 6-(1,2,4-triazolylmethyl)-8-(3-chlorophenyl)quinoline dihydrochloride (0.22 mmol, 18%) as light brown crystals, mp 208.1-208.5°C.

### PREPARATION OF 6-(IMIDAZOLYLMETHYL)-8-(3-CHLOROPHENYL)QUINOLINE

### Formula I, Where R¹ Is Imidazolylmethyl and R² Is 3-Chlorophenyl

6-Bromomethyl-8-(3-chlorophenyl)quinoline (1.7 mmol) was reacted with a solution of s-BuL₁ (1.7 mmol) and imidazole (1.87 mmol) in THF at -78°C, then room temperature for 1h to obtain 6-(imidazolylmethyl)-8-(3-chlorophenyl)quinoline dihydrochloride (1.35 mmol, 79 %),
mp 65-65.4°C.

### PREPARATION OF 6-(IMIDAZOLYLMETHYL)-8-(3-NITROPHENYL)QUINOLINE

### Formula I, Where R¹ Is Imidazolylmethyl and R² Is 3-Nitrophenyl

6-Bromomethyl-8-(3-nitrophenyl)quinoline (1.28 mmol) was reacted with a solution of n-BuLi (1.92 mmol) and imidazole (1.92 mmol) in THF at -78° C, then room temperature for 24 h to give 6-(imidazolylmethyl)-8-(3-nitrophenyl)quinoline (0.52 mmol, 40%), mp 129.5-130.5°C.

### EXAMPLE 15 (REACTION SCHEME C-1)

### PREPARATION OF 6-(4-FLUOROBENZYL)-8-(3-NITROPHENYL)QUINOLINE

### Formula I, Where R¹ Is 4-Fluorobenzyl and R² Is 3-Nitrophenyl

0 .5g (1.46 mmol) of 6-bromomethyl-8-(3-nitrophenyl)quinoline was dissolved in 15 mL of 1:1 ethanol/benzene. 0.41g ( 2.9 mmol) of 4-fluorophenyl boronic acid, 3 mL (5.8 mL) 2M Na₂CO₃, and 0.07g (0.06 mmol) Pd(PPh₃)₄ were added. The reaction mixture was refluxed under nitrogen for 20 min, cooled and poured into water (30 mL) and extracted with ethyl acetate (30 mL). The organic layer was washed with water (25 mL) and brine (25 mL), dried over MgSO₄, filtered and concentrated. The residue was purified by flash chromatography using 30% ethyl acetate/ hexane to obtain 0.41g of an impure yellow oil which was further purified by preparative TLC using the same solvent system. 0.33g of a clear oil was obtained which was crystallized from ethyl acetate/hexanes at 0° C to give 0.2g (0.56 mmol, 36%) of 6-(4-fluorobenzyl)-8-(3-nitrophenyl)quinoline as white crystals, mp 97.7-98.5°C.

### PREPARATION OF 6-(4-METHOXYBENZYL)-8-(3-NITROPHENYL)QUINOLINE

### Formula I, Where R¹ Is 4-Methoxybenzyl and R² Is 3-Nitrophenyl

6-Bromomethyl-8-(3-nitrophenyl)quinoline (1.17 mmol) was reacted with 4-methoxyboronic acid (2.34 mmol), 2M Na₂CO₃ (4.68 mmol), and Pd(PPh₃)₄ (0.05 mmol) to obtain 6-(4-methoxybenzyl)-8-(3-nitrophenyl)quinoline (0.49 mmol, 42%) as tan crystals, mp 117.5-119.1°C.

### EXAMPLE 16 (REACTION SCHEME B-1)

### PREPARATION OF 6-ISOPROPYL-8-(3,4-METHYLENEDIOXYPHENYL)QUINOLINE

### Formula I, Where R¹ Is Isopropyl and R² Is 3,4-Methylenedioxyphenyl

0.84g (3.36 mmol) of 6-isopropyl-8-bromoquinoline was dissolved in 15 mL of dry THF and cooled to -78°C. s-BuLi ( 2.7 mL, 3.53mmol, (1.3M/cyclohexane)) was added dropwise under nitrogen. The reaction mixture was stirred for 5 min and then ZnCl₂ (7 mL, 3.53 mmol, (0.5M/THF)) was added dropwise. The reaction mixture was warmed to room temperature over a period of 1 h. 0.44mL (3.7 mmol) of 1-Bromo-3,4-methylenedioxybenzene was added dropwise followed by 0.19g ( 0.17mmol) Pd(PPh₃)₄. The reaction mixture was refluxed for 2.5 h, cooled and poured into water (25 mL) and ethyl acetate (25 mL). The organic layer was washed with water (20 mL) and brine (20 mL), dried over MgSO₄, filtered and concentrated. The resulting brown oil was purified by flash chromatography using 15% ethyl acetate /hexanes to obtain 0.47g of a pure, clear oil which was crystallized from ethyl acetate/hexanes. Yield: 0.25g (0.85 mmol, 25%) of 6-isopropyl-8-(3,4-methylenedioxyphenyl)quinoline as white platelets, mp 86.6-88.2°C.

### PREPARATION OF 6-ISOPROPYL-8-(3-CYANOPHENYL)QUINOLINE

### Formula I, Where R¹ Is Isopropyl and R² Is 3-Cyanophenyl

6-Isopropyl-8-bromoquinoline (3.8 mmol) was reacted with 1.3M s-BuLi (3.99 mmol), ZnCl₂ (3.8 mmol), 3-cyanobromobenzene (3.8 mmol), and Pd(PPh₃)₄ (0.19 mmol) to obtain 6-isopropyl-8-(3-cyanophenyl)quinoline dihydrochloride (1.1 mmol, 29%) as white crystals, mp 194.8-197.6°C.

### PREPARATION OF 6-ISOPROPYL-8-(4-METHOXYPHENYL)QUINOLINE

### Formula I, Where R¹ Is Isopropyl and R² Is 4-Methoxyphenyl

6-Isopropyl-8-bromoquinoline (3 mmol) was reacted with 1.3M s-BuLi (3.03 mmol), ZnCl₂ (3 mmol), 4-methoxyiodobenzene (3.6 mmol), and Pd(PPh₃)₄ (0.15 mmol). 6-isopropyl-8-(4-methoxyphenyl)quinoline (0.67 mmol, 23%) was obtained as a clear oil.

### PREPARATION OF 6-ISOPROPYL-8-(4-TRIFLUOROMETHYLPHENYL)QUINOLINE

### Formula I, Where R¹ Is Isopropyl and R² Is 4-Trifluoromethylphenyl

6-Isopropyl-8-bromoquinoline (2.7 mmol) was reacted with 1.3M s-BuLi (2.83 mmol), ZnCl₂ (2.83 mmol), 4-trifluoromethyliodobenzene (4.05 mmol), and Pd(PPh₃)₄ (0.14 mmol) to obtain 6-isopropyl-8-(4-trifluoromethylphenyl)quinoline hydrochloride (0.97 mmol, 36 %) as off-white crystals, mp 167.5-168.4°C.

### EXAMPLE 17 (REACTION SCHEME B)

### PREPARATION OF METHYL 3-TRIFLUOROMETHANESULFOXYBENZOATE

### Formula 3C, Where R² Is Trifluoromethanesulfonyloxy

10g (65.73 mmol) of Methyl 3-hydroxybenzoate was dissolved in 330 mL of CH₂Cl₂ and cooled to 0°C. 45.8 mL (328.65 mmol) of triethylamine was added to form a light brown solution. Trifluoromethanesulfonic anhydride (triflic anhydride) ( 16.6 mL, 98.6 mmol) was added via addition funnel under nitrogen over 0.5h to form a dark brown solution. The reaction mixture was poured into a saturated solution of NaHCO3 (200 mL) and extracted with CH₂Cl₂ (200 mL). The organic layer was washed with water (2 x 100mL) and brine (100 mL), dried over MgSO₄, filtered, and concentrated. Flash chromatography using 20% ethyl acetate/ hexanes provided a slightly impure brown oil which was distilled (1.2 torr, 92-94.5°C) to yield 13.79g ( 48.52 mmol, 74%) of 3-(trifluoromethanesulfonyloxy)benzoic acid methyl ester (methyl 3-trifluoromethanesulfoxybenzoate) as a yellow liquid.

### PREPARATION OF 6-ISOPROPYL-8-(3-CARBOMETHOXYPHENYL)QUINOLINE

### Formula I, Where R¹ Is Isopropyl and R² Is 3-Methylbenzoate

6-Isopropyl-8-bromoquinoline (4.12 mmol) was reacted with 1.3M s-BuLi (4.33 mmol), ZnCl₂ (4.33 mmol), methyl 3-trifluorosulfoxybenzoate (4.33 mmol), and Pd(PPh₃)₄ (0.21 mmol) to obtain 6-isopropyl-8-(3-carbomethoxyphenyl)quinoline hydrochloride (0.56g, 1.64 mmol, 40%) which was crystallized from ethanol/ether/hexanes to yield light yellow crystals, mp 166.5-167.6°C.

### EXAMPLE 18 (REACTION SCHEME C-1)

### PREPARATION OF 6-CYANOMETHYL-8-(3-NITROPHENYL)QUINOLINE

### Formula I, Where R¹ Is Cyanomethyl and R² Is 3-Nitrophenyl

1g (2.91 mmol) of 6-Bromomethyl-8-(3-nitrophenyl)quinoline was dissolved in 5mL of CH₂Cl₂. To this solution was added dropwise 0.78g (2.91 mmol) of tetrabutylammonium cyanide dissolved in 5 ml of CH₂Cl₂ over 5 min. The reaction mixture was stirred for 1h at room temperature then refluxed overnight. An additional 0.39g (1.45 mmol) of tetrabutylammoniun cyanide was added and the reaction refluxed for 3 more hours. The reaction was then concentrated and ethyl acetate (30 mL) and water (30 mL) were added. The organic layer was washed with water (2 x 30 mL), and brine (30 mL). CH₂Cl₂ (50 mL) was added to the organic layer to solubilize the suspended solids and the solution was then dried over MgSO_{4,} filtered and concentrated. The residue was dissolved in a minimal amount of CH₂Cl₂, absorbed on silica gel, and placed on top of a silica gel column and eluted with 50% ethyl acetate/hexanes. Yield 0.6g (2.07 mmol, 71%) of 6-cyanomethyl-8-(3-nitrophenyl)quinoline as a yellow solid, mp 178.0-180.5°C.

### PREPARATION OF 6-[1-(4-CHLOROPYRIDAZINYL)-1-CYANOMETHYL]-8-(3-NITROPHENYL]QUINOLINE

### Formula I, Where R¹ Is [1-(4-Chloropyridazinyl)-1-cyanomethyl] and R² Is 3-Nitrophenyl

0.45g (1.56 mmol) of 6-cyanomethyl-8-(3-nitrophenyl)quinoline and 0.49 (3.28 mmol) of 1,4-dichloropyridazine were dissolved in 15 mL of dry DMF under nitrogen and cooled to 0 C. NaH (0.078g 3.28 mmol) was added portionwise and the reaction stirred at 0 C for 45 min and then 30 min at room temperature. The reaction mixture was poured into CH₂Cl₂ (150 mL) and 150 mL of half saturated NH₄Cl was added. The organic layer was separated and the aqueous layer was washed with CH₂Cl₂ (100 mL). The combined organic layers were washed with water (2 x 100 mL), and brine (100 mL), dried over MgSO₄, filtered and concentrated. Chromatography using 50% ethyl acetate/ hexanes yielded 0.51g (1.27 mmol, 81%) of 6-(1-(4-chloropyridazinyl)-1-cyanomethyl)-8-(3-nitrophenyl)quinoline as an orange foam.

### PREPARATION OF 6-PYRIDAZINONYLMETHYL)-8-(3-NITROPHENYL)QUINOLINE

### Formula I, Where R¹ Is Pyridazinonylmethyl] and R² Is 3-Nitrophenyl

0.4g (1 mmol) of 6-(1-(4-chloropyridazine)-1-cyanomethyl)-8-(3-nitrophenyl)quinoline was dissolved in 5 mL acetic acid, 5 mL water, and 10 mL conc. hydrochloric acid. The reaction mixture was refluxed for 24h, and then concentrated. The residue was dissolved in water (25 mL), saturated NaHCO₃ (150 mL) and ethyl acetate (100 mL) were added. The organic layer was washed with water (100 mL) and brine (100 mL), dried over MgSO₄, filtered and concentrated to obtain 0.27g of a brown solid. The residue was purified by preparative TLC using 5% MeOH/ CH₂Cl₂. Yield: 0.25g (0.7 mmol, 70%) of 6-(4-pyridazinonylmethyl)-8-(3-nitrophenyl)quinoline as an off-white solid, mp 199.5-202.0° C.

### EXAMPLE 19 (REACTION SCHEMES A-1, B)

### PREPARATION OF 4-NITRO-1-CYCLOPENTYLIDINYLBENZENE

### Formula 1a, Where R Is Cyclopentyl

5g (33.42 mmol) of Cyclopentyltriphenylphosphine bromide was slurried in 100mL of dry THF and 18.4 mL of 1.8M PhLi ( 33.09 mmol) was added over 5 min. The reaction mixture was stirred for 1h at room temperature, then refluxed for 15 min, and cooled to room temperature. 5g (33.09 mmol) of 4-nitrophenyl carboxaldehyde in 40 mL of dry THF was added dropwise. The reaction mixture was stirred at 40° C for 24 h, then poured into water (100 mL) and ethyl acetate (100 mL). The aqueous layer was extracted with ethyl acetate (3 x 50 mL) . The combined organic layers were dried over MgSO₄, filtered, and concentrated. Flash chromatography using 50% ethyl acetate/hexanes yielded 4.7 g (23.13 mmol) of 4-nitro-1-cyclopentylidinylbenzene as an impure black oil.

### PREPARATION OF CYCLOPENTYLIDINYLANILINE

### Formula 1b, Where R Is Cyclopentyl

4.7g of 4-nitro-1-cyclopentylidinylbenzene was dissolved in ethanol (100 mL) and catalytically hydrogenated (Pd/C) under 1 atm of hydrogen for 24h. The reaction mixture was filtered through Celite, concentrated to an orange semi-solid, and chromatographed using 10% ethyl acetate to obtain 2.14g (12.35 mmol) of 4-cyclopentylidinylaniline as an yellow semi-solid.

### PREPARATION OF 4-CYCLOPENTYLMETHYLANILINE

### Formula 2a, Where R Is Cyclopentyl

1.83g (10.32 mmol) of 4-cyclopentylidinylaniline was dissolved in ethanol (50 mL) and catalytically hydrogenated (PtO₂) under 1 atm of hydrogen for 30 min. The reaction mixture was filtered through Celite and concentrated. The residue was chromatographed using 20% ethyl acetate/ hexanes to afford 1.52 g of a yellow oil as a mixture of starting material and product. The yellow oil was dissolved in ethanol (25 mL) and catalytically hydrogenated (PtO₂) under 1 atm of hydrogen for 5h. Filtration through Celite, concentration, and chromatography using 20% ethyl acetate/ hexanes gave 1.52g (8.67 mmol 84%) of 4-cyclopentylmethylaniline as a clear oil.

### PREPARATION OF 2-BROMO-4-CYCLOPENTYLMETHYLANILINE

### Formula 3a, Where R Is Cyclopentyl and X is Bromo

1.07g (6.1 mmol) of 4-cyclopentylmethylaniline was dissolved in DMF (2.5 mL) and placed under nitrogen. 1.09g (6.1 mmol) of N-bromosuccinamide, dissolved in DMF (2.5 mL), was added dropwise.
The reaction mixture was stirred for 5h, then poured into water (100 mL), and extracted with ether (3 x 25 mL). The combined ether extracts were dried over MgSO₄ and filtered. Concentration and flash chromatography on silica gel using 10% ethyl acetate/hexanes provided 0.98g (3.86 mmol, 63%) of 2-bromo-4-cyclopentylmethylaniline as a light brown oil.

### PREPARATION OF 6-CYCLOPENTYLMETHYL-8-BROMOQUINOLINE

### Formula 5, Where R Is Cyclopentylmethyl and X is Bromo

0 .66g (2.6 mmol) of 2-bromo-4-cyclopentylmethylaniline was slurried in 0.094g (0.34 mmol) Iron (II) sulfate heptahydrate, 0.17 mL (1.66mmol) nitrobenzene, and 1g (10.4 mmol) of glycerol, and heated to 115°C under nitrogen. H₂SO₄ (0.55 mL) was added dropwise and the reaction mixture was heated to 165°C, stirred for 2h and then poured into saturated NaHCO₃ (300 mL) and ethyl acetate (50 mL). The solution was filtered through glass wool and the filtrate was extracted with ethyl acetate (2 x 50mL). The combined extracts were dried over MgSO₄ and filtered. Concentration and flash chromatography (10% EtOAc/hexanes) yielded 0.58g (2 mmol, 77%) of 6-cyclopentylmethyl-8-bromoquinoline as a yellow oil.

### PREPARATION OF 6-CYCLOPENTYLMETHYL-8-(3-CHLOROPHENYL)QUINOLINE

### Formula I, Where R¹ Is Cyclopentylmethyl and R² is 3-Chlorophenyl

0.19g (0.65 mmol) of 6-Cyclopentylmethyl-8-bromoquinoline was dissolved in 6.5 mL of 1:1 ethanol/ benzene under nitrogen. 0.2g (1.3 mmol) of 3-chloroboronic acid, 1.3 mL (2.6 mmol) of 2M Na₂CO₃, and 0.03g (0.026 mmol) of Pd(PPh₃)₄ were added successively. The reaction mixture was refluxed for 3h, then cooled and poured into water (25 mL) and ethyl acetate (25 mL). The organic extract was dried over MgSO₄ and filtered. Flash chromatography on silica gel using 20% ethyl acetate/hexanes yielded 0.17g of impure product which was purified further by preparative TLC in the same solvent system to obtain 0.16g yellow oil. Formation of the HCl salt with saturated HCl/EtOAc and crystallization of the resulting residue from EtOH/EtOAc/ether yielded 0.14 g (0.4 mmol, 60%) of 6-cyclopentylmethyl-8-(3-chlorophenyl)quinoline hydrochloride as white crystals, mp 179.3-183.7°C.

### PREPARATION OF 6-CYCLOPENTYLMETHYL-8-(3-NITROPHENYL)QUINOLINE

### Formula I, Where R¹ Is Cyclopentylmethyl and R² is 3-Nitrophenyl

Similarly, 6-Cyclopentylmethyl-8-bromoquinoline (0.55 mmol) was reacted with 3-chloroboronic acid (1.1 mmol), 2M Na₂CO₃ (2.2 mmol), and Pd(PPh₃)₄ (0.02 mmol) to obtain 6-cyclopentylmethyl-8-(3-nitrophenyl)quinoline hydrochloride (0.42 mmol, 77 %), as tan crystals from EtOH/EtOAc/Et₂O, mp 185.8-190.4°C.

### EXAMPLE 20 (REACTION SCHEMES B-3, B)

### PREPARATION OF 6-METHOXY-8-AMINOQUINOLINE

### Formula 5, Where R¹ Is Methoxy and X is Amino

4 g (19.6 mmol) of commercially available 6-methoxy-8-nitroquinoline was dissolved in ethanol (100 mL) and THF (25 mL) and catalytically hydrogenated (Pd/C, 1 atm H2) for 5h. The reaction mixture was filtered through Celite and concentrated. Flash chromatography using 5% MeOH/ CH₂Cl₂ yielded an impure product which was re-chromatographed in 50% EtOAc/hexanes to give 2.76g (15.84 mmol, 81%) of 6-methoxy-8-aminoquinoline as a yellow oil.

### PREPARATION OF 6-METHOXY-8-BROMOQUINOLINE

### Formula 5, Where R¹ Is Methoxy and X is Bromo

1.25 g (7.18 mmol) of 6-methoxy-8-aminoquinoline was mixed with 48% HBr (6 mL) and water (6 mL) to form an orange slurry. The mixture was then cooled to 0C and a solution of 5 g of NaNO₂ (9.3 mmol) in 5 mL of water was added dropwise. The resulting brown solution was stirred for 15 min and then added to a solution of 1.24 g CuBr (8.6 mmol) in 15 mL of 48% HBr with stirring at 75°C. The reaction mixture was stirred at 75°C for 5.5h and was then made basic with 10% NaOH and filtered through filter paper. The filtrate was partitioned between water (50 mL) and EtOAc (50 mL) and extracted with EtOAc (2 x 50 mL). The combined organic extracts were dried over MgSO₄, filtered, and concentrated to give 0.6 g of a dark oil which was purified by preparative TLC (20% EtOAc/hexanes) to afford 0.36g (1.5 mmol, 21%) of 6-methoxy-8-bromoquinoline as a tan solid, mp 64.5-65.6°C.

### PREPARATION OF 6-HYDROXY-8-BROMOQUINOLINE

### Formula 5, Where R¹ Is Hydroxy and X is Bromo

0.26g ( 1.1 mmol) of 6-methoxy-8-bromoquinoline was dissolved in CH₂Cl₂ (11 mL) and cooled to -78°C. 2.7 mL (2.75 mmol) of BBr₃ (1M in CH₂Cl₂) was added dropwise to form a yellow slurry. The reaction mixture was stirred for 10 min at -78°C, the bath was removed and the reaction mixture was allowed to warm to room temperature, stirred for 1.5 h and then MeOH (20 mL) was added to quench the reaction. The reaction mixture was concentrated under reduced pressure, 20 mL of MeOH was added and again concentrated (repeated 2x). The residue was partitioned between EtOAc and water. The organic layer was washed 2x with NaOH (0.5 N). The base extracts were neutralized with saturated NH₄Cl and extracted with EtOAc (2 x 25 mL). The organic layers were washed with brine, dried over MgSO₄, filtered, and concentrated to yield 0.llg (0.49 mmol, 45 %) of 6-hydroxy-8-bromoquinoline as an off-white solid, mp 249.5-250.6°C.

### PREPARATION OF 6-CYCLOPENTYLOXY-8-BROMOQUINOLINE

### Formula 5, Where R¹ Is Cyclopentyloxy and X is Bromo

0.2g (0.89 mmol) of 6-hydroxy-8-bromoquinoline was dissolved in DMF (5 mL). 0.25g (1.78 mmol) of powdered K₂CO₃ and 0.2 mL(1.78 mmol) cyclopentylbromide were added, and reaction mixture was heated at 75°C for 1.5h. The reaction mixture was then cooled and poured into water (20 mL) and extracted with ether (20 mL). The ether layer was washed with water (30 mL) and brine (30 mL). The aqueous layers were re-extracted with ether (20 mL) and the combined organic layers were dried over MgSO₄, filtered and concentrated to obtain 0.26g (0.89 mmol, 100%) of 6-cyclopentyloxy 8-bromoquinoline as a crude yellow solid, mp 76.2-79.2°C.

### PREPARATION OF 6-CYCLOPENTYLOXY-8-(3-NITROPHENYL)QUINOLINE

### Formula I, Where R¹ Is Cyclopentyloxy and R² is 3-Nitrophenyl

0.22g (0.75 mmol) of 6-cyclopentyloxy-8-bromoquinoline was dissolved in 7.5 mL of 1:1 EtOH/benzene and 0.25g (1.5 mmol) 3-nitroboronic acid, 1.5 mL (3 mmol) 2M Na₂CO₃, and 0.035g (0.3 mmol) Pd(PPh₃)₄ were added successively. The reaction mixture was refluxed for 2h under nitrogen, then partitioned between water/EtOAc. The organic layer was washed with water, brine, dried over MgSO₄, and filtered. Concentration and purification by preparative TLC using 15% EtOAc/hexanes provided 0.2g of a yellow oil. The hydrochloride salt was formed with saturated HCl/EtOAc and crystallized from EtOH/EtOAc/ether. Yield: 0.21g (0.56 mmol, 75%) of 6-cyclopentyloxy-8-(3-nitrophenyl)quinoline as white crystals, mp 190-191.4°C.

### EXAMPLE 21

By following the procedures in Examples 1-20, other 6,8-disubstituted quinolines within the scope of this invention can be prepared.
For example, the following 6,8-disubstituted quinoline compounds of formula I were prepared by following the above described procedures:
6-isopropyl-8- (3-nitrophenyl) quinoline, (hydrochloride),
   mp 204.5-216 °C;
4-pyridylmethyl-8-(3-nitrophenyl) quinoline, (dihydrochloride),
   mp 238-240°C;
6-[1-(1,2,4-triazolyl)]-8-(3-nitrophenyl)quinoline, mp 160.7-162.5°C;
6-anilinomethyl-8-(3-nitrophenyl)quinoline, mp 149.9-150.5°C;
6-(4-pyridazinonylmethyl)-8-(3-nitrophenyl)quinoline, mp 199.5-202°C;
6-(2-pyrrolidinonylmethyl)-8-(3-nitrophenyl)quinoline, mp 127.4-128.3°C.
6-cyclopentylmethyl-8-(3-nitrophenyl)quinoline, (hydrochloride),
   mp 186.3-191.2°C.
6-imidazolylmethyl-8-(3-nitrophenyl)quinoline, mp 129.5-130.5°C.
6-cyclohexyl-8- (3-nitrophenyl)quinoline, (hydrochloride),
   mp 188.5-194.8°C.
6-cyclopentyloxy-8-(3-nitrophenyl)quinoline, (hydrochloride),
   mp 190-191.4°C.
6-ethyl-8-(3-nitrophenyl)quinoline, (hydrochloride),
   mp 193.1-193.6°C.
6-n-propyl-8-(3-nitrophenyl) quinoline, (hydrochloride),
   mp 208.5-209.7°C.
6-n-butyl-8-(3-nitrophenyl)quinoline, (hydrochloride),
   mp 194.1-198.5°C.
6-t-butyl-8-(3-nitrophenyl)quinoline, (hydrochloride),
   mp 214-214.8°C;
6-n-pentyl-8-(3-nitrophenyl)quinoline, (hydrochloride),
   mp 175.9-177.90C;
6-(4-fluorobenzyl)-8-(3-nitrophenyl)quinoline, mp 97.7-98.5°C.
6-pyrrolidinyl-8-(3-nitrophenyl)quinoline, (dihydrochloride)
   mp 223-225°C.
4-pyridylmethyl-8-(3-chlorophenyl)quinoline, (dihydrochloride),
   mp 243.0-246.4°C;
6-[1-(1,2,4-triazolyl)]-8-(3-chlorophenyl)quinoline, (dihydrochloride),
   mp 208.1-208.5°C;
6-imidazolylmethyl-8-(3-chlorophenyl)quinoline, (dihydrochloride),
   mp 64-64.5°C.
6-ethyl-8-(3-chlorophenyl)quinoline, (hydrochloride),
   mp 184.8-185.9°C.
6-n-propyl-8-(3-chlorophenyl)quinoline, (hydrochloride),
   mp 206.4-210.4°C.
6-n-butyl-8-(3-chlorophenyl)quinoline, (hydrochloride),
   mp 202.3-208.7°C.
6-t-butyl-8-(3-chlorophenyl)quinoline, (hydrochloride),
   mp 197.5-198.5°C;
6-n-pentyl-8-(3-chlorophenyl)quinoline, (hydrochloride),
   mp 182.2-183.6°C;
6-cyclohexyl-8-(3-chlorophenyl)quinoline, (hydrochloride),
   mp 197.7-203.7°C.
6-(1-hydroxy-1-methylethyl)-8-(3-chlorophenyl)quinoline, (hydrochloride),
   mp 171.2-171.9°C.
6-isopropyl-8-(3,4-methylenedioxyphenyl)quinoline, (hydrochloride),
   mp 86.6-88.2°C.
6-isopropyl-8-(3-carbomethoxyphenyl)quinoline, (hydrochloride),
   mp 166.5-167.6°C.
6-n-propyl-8-phenylquinoline, (hydrochloride), mp 168-169°C.
6-isopropyl-8-phenylquinoline, (hydrochloride), mp 155-158.9°C.
6-n-pentyl-8-phenylquinoline, (hydrochloride), mp 151.5-152.3°C.
6-isopropyl-8-(4-trifluoromethylphenyl)quinoline, (hydrochloride),
   mp 167.5-168.6°C.
6-isopropyl-8-(3-cyanophenyl)quinoline, (hydrochloride),
   mp 206.5-207.6°C.

### EXAMPLE 22

### Determination of Potency and Selectivity of Inhibitors for PDE IV

### Preparation of Human Platelet Phosphodiesterase (PDE III)

Platelet high-affinity cAMP PDE (PDE III) was obtained from human blood in accordance with previously described procedures described in *Mol. Pharmacol.* 20:302-309, Alvarez, R., Taylor, A., Fazarri, J.J., and Jacobs, J.R. (1981).

Blood was collected into evacuated tubes containing EDTA (7.7 mM, final concentration). PRP was obtained by centrifuging the blood in polycarbonate tubes at 200 x g for 15 min at 4°C. A platelet pellet was resuspended in a volume of buffer A (0.137 M NaCl, 12.3 mM Tris-HCl buffer, pH 7.7, containing 1 mM MgCl₂. The hypotonically-lysed platelet suspension was centrifuged at 48,000 x g for 15 min and the supernatant was saved. The pellets were frozen on dry ice and briefly thawed at 22°C. The supernatant was combined with the pellet fraction and the resulting suspension was centrifuged at 48,000 x g for 30 min. The supernatant fraction was stored in 0.5 mL aliquots at -20°C and used as the soluble PDE. Enzyme activity was adjusted to 10-20% hydrolysis after 10 minutes of incubation by dilution with 10mM cold Tris-HCl buffer, pH7.7.

### Preparation of Human Lymphocyte Phosphodiesterase (PDE IV)

Human B cell line (43D) were cultured at 37°C in 7% CO₂ in RPMI 1640 with L-glutamine and 10% Nu-Serum. Prior to the assay ∼1.5x10⁸ cells were centrifuged at 1000 rpm for 10 minutes in a table top clinical centrifuge. The pellet was resuspended in 2-3 mL of 45 mM Tris-HCl buffer, pH 7.4. The suspension was homogenized and centrifuged at 12,000 x g at 4°C for 10 minutes. The supernatant was diluted to 28 mL with Tris-HCl buffer and used directly in the assay or stored at -20°C. The final concentration of DMSO in the PDE incubation medium was 1%. Nitraquazone was included in each assay (10 and 100µM) as a reference standard.

### Human Platelet cAMP Phosphodiesterase Assay

The phosphodiesterase incubation medium contained 10 mM Tris-HCl buffer, pH 7.7, 10 mM MgSO₄, 0.1-1µM [³H]-AMP (0.2 µCi) in a total volume of 1.0 mL. Following addition of the enzyme, the contents were mixed and incubated for 10 min at 30°C. The reaction was terminated by immersing the tubes in a boiling-water bath for 90 sec. After the tubes were cooled in an ice-water bath, 0.1 mL (100µg) of 5'-nucleotidase from snake venom (Crotalus atrox, Sigma V-7000) was added to each tube. The contents were mixed and incubated for 30 min at 30°C. The nucleotidase reaction was terminated by immersing the tubes in a boiling water bath for 60 sec. Labeled adenosine was isolated from alumina columns according to the method described *in Anal. Biochem.,* **52**:505-516 (1973), Filburn, C.R., and Karn, J.. Assays were performed in triplicate. Hydrolysis of cAMP ranged from 10-20%. Test compounds were dissolved in DMSO. The final concentration of DMSO in the phosphodiesterase assay was 1% when tested with compounds up to 0.1 mM. When tested at 1 mM the DMSO concentration was 10% and this activity was compared to control PDE activity in the presence of 10% DMSO.

| **PDE III Activity** | | |
|---|---|---|
| Compound I | IC₅₀ | 53 µM |

**Compound I** is 6-(4-pyridylmethyl)-8-(3-nitrophenyl)quinoline.

### Human Lymphocyte cAMP Phosphodiesterase Assay

The phosphodiesterase incubation medium contained 40 mM Tris-HCl buffer, pH 7.7, 0.1 mM MgSO₄, 3.75 mM mercaptoethanol, and 0.1-1.0 µM [³H] cAMP (0.2 µCi) in a total volume of 1.0 mL. The reaction was performed and processed according to the procedure used (above) for human platelet PDE. The final concentration of DMSO was 1%.

The representative compounds of the present invention exhibit potency and selectivity as inhibitors of PDE IV when tested by the human platelet cAMP phosphodiesterase assay and the human lymphocyte cAMP phosphodiesterase assay.

| **Inhibition of Human Lymphocyte PDE IV** | | |
|---|---|---|
| Compound I | IC₅₀ | 0.023 nM |
| Compound II | IC₅₀ | 0.11 nM |
| Compound III | IC₅₀ | 0.063 nM |

**Compound I** is the compound of Formula I where R¹ is 4-pyridylmethyl and R² is 3-nitrophenyl, namely 6-(4-pyridylmethyl)-8-(3-nitrophenyl)quinoline.

**Compound II** is the compound of Formula I where R¹ is 4-pyridylmethyl and R² is 3-chlorophenyl, namely 6-(4-pyridylmethyl)-8-(3-chlorophenyl)quinoline.

**Compound III** is the compound of Formula I where R¹ is isopropyl and R² is 3-nitrophenyl, namely 6-isopropyl-8-(3-nitrophenyl)quinoline.

### EXAMPLE 23

### Determination of Immunosuppressive Activity Utilizing Responses of Human Peripheral Blood Lymphocytes to Mitogen

This procedure is a modification of a procedure initially described by Greaves, et al. ["Activation of human T and B lymphocytes by polyclonal mitogens," *Nature,* 248, 698-701 (1974)].

Human mononuclear cells (PBL) were separated from heparinized whole blood by density gradient centrifugation in Ficoll-Paque (Pharmacia). After washing, 5 x 10⁴ cells/well were cultured in microtiter plates with minimal essential media supplemented with 1% human serum, gentamicin, sodium bicarbonate, 2-mercaptoethanol, glutamine, non-essential amino acids, and sodium pyruvate. The mitogen concanavalin A (Sigma) was used at a concentration of 2 µg/ml. Test materials were tested at concentrations between 10⁻⁴ and 10⁻¹⁰ M, by addition to the culture at time 0. Cultures were set up in quadruplicate and incubated at 37°C in a humidified atmosphere with 5% CO₂ for 48 hours. A pulse of 1.0 µCi/well of ³H-thymidine was added for the last 4 hours. Cells were collected on glass fiber filters with an automatic harvester and radioactivity was measured by standard scintillation procedures. The 50% inhibitory concentration ("IC₅₀") for mitogenic stimulation was determined graphically.

The representative compounds of the present invention showed immunosuppressive activity when tested by this method.

### EXAMPLE 24

### Determination of Immunosuppressive Activity Utilizing The Hemolytic Plaque Forming Cell Assay

This procedure is a modification of "The agar plaque technique for recognizing antibody producing cells," a procedure initially described by Jerne, et al. [*Cellbound Antibodies,* Amos and Kaprowski editors (Wistar Institute Press, Philadelphia, 1963) p. 109].

Groups of 5-6 adult C3H female mice were sensitized with 1.25 x 10⁸ sheep red blood cells (SRBC) and simultaneously treated with an oral dosage form of the test material in an aqueous vehicle. Animals in a control group received the same volume of vehicle. Four days after SRBC inoculation, spleens were dispersed in glass homogenizers. The number of nucleated cells (WBC) was determined and the spleen cell suspension was mixed with SRBC, guinea pig complement and agar solution at 0.5% concentration. Aliquots of the above mixture (0.1 mL) were dropped on four separate quadrants of a Petri dish and were covered with cover slips. After two hours incubation at 37°C, areas of hemolysis around plaque-forming cells (PFC) were counted with a dissecting microscope. Total WBC/spleen, PFC/spleen and PFC/10⁶ WBC (PPM) were calculated for each mouse spleen. Geometric means of each treatment group were then compared with the vehicle-treated control group.

The representative compounds of the present invention showed immunosuppressive activity when tested by this method.

### EXAMPLE 25

### Determination of Anti-Inflammatory Activity Utilizing Arachidonic Acid-Induced Ear Edema (AAEE) in the Mouse

This procedure is a modification of a procedure described by Young et al., J. *Invest. Derm.,* 82:367-371 (1984).

Female Charles River ICR mice 23-27 grams were administered 0.2 mL of test material. The mice were challenged with 20 µl of arachidonic acid applied topically to the ear. One hour after challenge, the weight of an 8 mm disc was determined. The mean increase in ear plug weight was calculated.

Materials with anti-inflammatory activity inhibit the increase in ear plug weight.

The representative compounds of the present invention exhibited anti-inflammatory activity when tested by this method.

| **Anti-inflammatory Activity (AAEE)** | | |
|---|---|---|
| Compound I | ED₅₀ | 0.01mg/kg |
| Compound II | ED₅₀ | 0.2mg/kg |
| Compound III | ED₅₀ | 4.5mg/kg |

### EXAMPLE 26

### Determination of Anti-Inflammatory Activity Utilizing Adjuvant-Induced Arthritis In The Rat

This procedure is a modification of a procedure initially described by Pearson, C.M., *Proc. Soc. Exp. Biol. Med.,* 91:95-101 (1956).

Female Charles River albino rats weighing 160-180 g received 0.1 mL of a suspension in paraffin oil of heat-killed *Mycobacterium butyricum* (10 mg/ml) by means of an intradermal injection into the proximal 1/4 of the tail on day 0. Beginning on day 1, the test material was administered orally in an aqueous vehicle (0.5 mL/dose) once each day for 17 days. On day 18 the intensity of the swelling of the four foot pads and tail was determined utilizing a scoring system in which the swelling in the four paws was scored 0-4 for each paw and the tail swelling was scored 0-3, such that the total maximum score was 19.

The representative compounds of the present invention exhibited anti-inflammatory activity when tested by this method.

### EXAMPLE 27

### Determination of Activity Towards Autoimmune Disease Utilizing Survival of MRL/lpr Mice

MRL/lpr mice develop a multisystemic disease characterized by glomerulonephritis, arthritis, arteritis, lymphoid hyperplasia. The length of survival of mice with this disease is approximately one-third that of non-disease developing MRL/n mice. These mice have a high incidence of autoantibodies and the disease process is considered autoimmune in nature as described by Theofilopoulos, et al., *Advances in Immunology,* 37:269-390 (1985).

The representative compounds of the present invention significantly extended the lifespan of the MRL/lpr mice.

### EXAMPLE 28

### Determination of Analgetic Activity Utilizing Phenylquinone-Induced Stretching in the Mouse

This procedure is a modification of a procedure described by Hendershot, et al. *J*. *Pharmacol. Exp. Ther., 125*:237-240 (1959).

Groups of 8 Female CD-1 mice were administered test materials orally in an aqueous vehicle. At various times following administration of test materials, 0.25 mL of a 0.02k solution of phenylquinone was administered intraperitoneally. The number of stretches for each animal was enumerated over a ten minute period following the phenylquinone administration. Analgetic activity was determined by inhibition of the mean number of stretches.

The representative compounds of the present invention showed analgetic activity when tested by this method.

### EXAMPLE 29

### Capsule Formulation

This example illustrates the preparation of a representative pharmaceutical formulation for oral administration containing an active compound of formula I wherein R¹ and R² are defined as above and additionally wherein R¹ can be hydrogen or formyl e.g., 6-isopropyl-8-(3-nitrophenyl)quinoline.

| **Ingredients** | **Quantity (mg/capsule)** |
|---|---|
| Active compound | 200 |
| lactose, spray-dried | 148 |
| magnesium stearate | 2 |

The above ingredients are mixed and introduced into a hard-shell gelatin capsule.

compound of formula I wherein R¹ and R² are defined as above and additionally wherein R¹ can be hydrogen or formyl, such as those prepared in accordance with Examples 1-20 can be used as the active compound in the preparation of the orally administrable formulations of this example.

### EXAMPLE 30

### Oral Formulation

This example illustrates the preparation of a representative pharmaceutical formulation containing an active compound of formula I wherein R¹ and R² are defined as above and additionally wherein R¹ can be hydrogen or formyl e.g., 6-isopropyl-8-(3-nitrophenyl)quinoline.

An suspension for oral administration is prepared having the following composition:

| **Ingredients** | **Quantity** |
|---|---|
| Active compound | 1.0 g |
| fumaric acid | 0.5 g |
| sodium chloride | 2.0 g |
| methyl paraben | 0.1 g |
| granulated sugar | 25.5 g |
| sorbitol (70k solution) | 12.85 g |
| Veegum K (Vanderbilt Co.) | 1.0 g |
| flavoring | 0.035 mL |
| colorings | 0.5 mg |
| distilled water | q.s. to 100 mL |

compound of formula I wherein R¹ and R² are defined as above and additionally wherein R¹ can be hydrogen or formyl, such as those prepared in accordance with Examples 1-20 can be used as the active compound in the preparation of the orally administrable formulations of this example.

### EXAMPLE 31

### Tablet Formulation

This example illustrates the preparation of a representative pharmaceutical formulation containing an active, compound of formula I wherein R¹ and R² are defined as above and additionally wherein R¹ can be hydrogen or formyl, e.g., 6-isopropyl-8-(3-nitrophenyl)quinoline.

A tablet for oral administration is prepared having the following composition:

| **Ingredients** | **Quantity (mg/tablet)** |
|---|---|
| Active compound | 400 |
| corn starch | 50 |
| lactose | 145 |
| magnesium stearate | 5 |

The above ingredients are mixed intimately and pressed into single scored tablets.

compound of formula I wherein R¹ and R² are defined as above and additionally wherein R¹ can be hydrogen or formyl, such as those prepared in accordance with Examples 1-20 can be used as the active compound in the preparation of the tablet formulations of this example.

### EXAMPLE 32

### Injectable Formulation

This example illustrates the preparation of a representative pharmaceutical formulation containing an active compound of formula I wherein R¹ and R² are defined as above and additionally wherein R¹ can be hydrogen or formyl, e.g., 6-isopropyl-8-(3-nitrophenyl)quinoline.

An injectable preparation is prepared having the following composition:

| **Ingredients** | **Quantity** |
|---|---|
| Active compound | 0.2 g |
| water (distilled, sterile) | q.s. to 20.0 mL |

compound of formula I wherein R¹ and R² are defined as above and additionally wherein R¹ can be hydrogen or formyl such as those prepared in accordance with Examples 1-20 can be used as the active compound in the preparation of the injection administrable formulations of this example.

### EXAMPLE 33

### Suppository Formulation

This example illustrates the preparation of a representative pharmaceutical formulation containing an active compound of formula I wherein R¹ and R² are defined as above and additionally wherein R¹ can be hydrogen or formyl, e.g., 6-isopropyl-8-(3-nitrophenyl) quinoline.

A suppository totalling 2.5 grams is prepared having the following composition:

| **Ingredients** | **Quantity** |
|---|---|
| Active compound | 500 mg |
| Witepsol H-15* | q.s. to 2.5 g |

| | |
|---|---|
| (*triglycerides of saturated vegetable fatty acid; a product of Riches-Nelson, Inc., New York, N.Y.). | |

compound of formula I wherein R¹ and R² are defined as above and additonally wherein R¹ can be hydrogen or formyl, such as those prepared in accordance with Examples 1-20 can be used as the active compound in the preparation of the suppository formulations of this example.

### TOXICOLOGY

No serious toxicological effects were observed in the above described assays.

## Claims

1. A compound of the formula 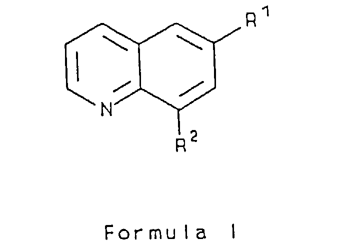 wherein:
R¹ is selected from C₁₋₄-alkyl, C₃₋₆-cycloalkyl, C₃₋₆-cycloalkyloxy, C₃₋₆-cycloalkylamino, C₃₋₆-cycloalkyl-C₁₋₄-alkyl, C₁₋₄-alkoxy, hydroxy-C₁₋₄-alkyl, carboxy-C₁₋₄-alkyl, aryl, aryloxy, arylamino, aryl-C₁₋₄-alkyl, heterocycle, heterocycle-oxy, heterocycle-amino and heterocycle-C₁₋₄-alkyl,
where cycloalkyl is optionally substituted with hydroxy, amino, imino, C₁₋ ₄-alkyl, C₁₋₄-alkoxy, carboxy, C₁₋₄-alkoxycarbonyl, carbamoyl, C₁₋₄-acyl, aryl, halo, and/or cyano;
where aryl represents a carbocyclic aromatic radical having 6 or 10 carbon atoms, which can optionally be mono-, di-, or tri-substituted, independently, with hydroxy, thiol, amino, halo, nitro, C₁₋₄-alkyl, C₁₋₄-alkylthio, C₁₋₄-alkyloxy, mono-C₁₋₄-alkylamino, di-C₁₋₄-alkylamino, carboxy, C₁₋₄- alkoxycarbonyl, hydroxysulfonyl, C₁₋₄-alkoxysulfonyl, C₁₋₄-alkylsulfonyl, C₁₋₄-alkylsulfinyl, trifluoromethyl, cyano, tetrazolyl, carbamoyl, C₁₋₄-alkyl-carbamoyl or di-C₁₋₄-alkylcarbamoyl;
where heterocycle represents a saturated, unsaturated or aromatic cyclic radical having at least one heteroatom, selected from nitrogen, oxygen or sulfur and is optionally substituted, independently, with hydroxy, amino, imino, C₁₋₄-alkyl, C₁₋₄-alkoxy, carboxy, C₁₋₄-alkoxycarbonyl, carbamoyl, C₁₋₄-acyl, aryl, halo, and/or cyano;
and
R² is phenyl, unsubstituted or independently mono-, di-, tri-, or tetra-substituted with C₁₋₄-alkyl, hydroxy, thiol, amino, halo, nitro, cyano, C₁₋₄-alkoxy, C₁₋₄-alkylthio, mono- or di-C₁₋₄-alkylamino, carboxy, C₁₋₄-alkoxycarbonyl, C₁₋₄-alkylene-dioxy, hydroxysulfonyl, C₁₋₄-alkoxysulfonyl, C₁₋₄-alkylsulfonyl, C₁₋₄-alkylsulfinyl, trifluoromethyl, trifluoromethyloxy, tetrazolyl, carbamoyl, and mono-or di-C₁₋₄-alkylcarbamoyl; provided that when R¹ is methoxy, R² is not 4-aminophenyl or 4-nitrophenyl;
or pharmaceutically acceptable salts or N-oxides thereof.

2. The compound of Claim 1 wherein R² is phenyl substituted in the 3-position with halo, nitro, cyano, C₁₋₄-alkoxycarbonyl or trifluoromethyl.

3. The compound of Claim 2 wherein R² is 3-chlorophenyl, 3-nitrophenyl or 3-cyanophenyl.

4. The compound of Claim 3 wherein R¹ is pyridylmethyl, benzyl, C₁₋₄-alkyl, hydroxy-C₁₋₄-alkyl, or C₃₋₆-cycloalkylmethyl.

5. The compound of Claim 4 wherein R¹ is 4-pyridylmethyl, benzyl, ethyl, n-propyl, isopropyl, n-butyl, 1-hydroxy-1-methylethyl, cyclopropylmethyl or cyclopentylmethyl.

6. The compound of Claim 1 wherein R² is phenyl substituted in the 3-and 4-position, independently, with halo, nitro, cyano, C₁₋₄-alkoxycarbonyl, C₁₋₄-alkylenedioxy or trifluoromethyl.

7. The compound of Claim 6 wherein R² is 3-chloro-4-fluorophenyl or 3,4-methylenedioxyphenyl.

8. The compound of Claim 7 wherein R¹ is pyridylmethyl, benzyl, C₁₋₄-alkyl, hydroxy-C₁₋₄-alkyl, or C₃₋₆-cycloalkylmethyl.

9. The compound of Claim 8 wherein R¹ is 4-pyridylmethyl, benzyl, ethyl, n-propyl, isopropyl, n-butyl, 1-hydroxy-1-methylethyl, cyclopropylmethyl or cyclopentylmethyl.

10. A pharmaceutical composition comprising a pharmaceutically acceptable excipient and a therapeutically effective amount of a compound as defined in Claim 1.

11. Use of a compound represented by the formula 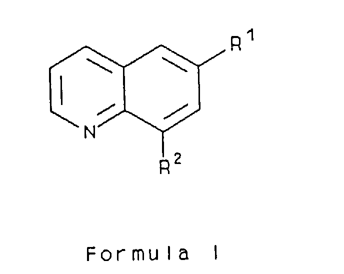 wherein:
R¹ is selected from hydrogen, C₁₋₄-alkyl, C₃₋₆-cycloalkyl, C₃₋₆-cycloalkyloxy, C₃₋₆-cycloalkylamino, C₃₋₆-cycloalkyl-C₁₋₄-alkyl, C₁₋₄-alkoxy, formyl, hydroxy-C₁₋₄-alkyl, carboxy-C₁₋₄-alkyl, aryl, aryloxy, arylamino, aryl-C₁₋₄-alkyl, heterocycle, heterocycle-oxy, heterocycle-amino and heterocycle-C₁₋₄-alkyl,
where cycloalkyl is optionally substituted with hydroxy, amino, imino, C₁₋₄-alkyl, C₁₋₄-alkoxy, carboxy, C₁₋₄-alkoxycarbonyl, carbamoyl, C₁₋₄-acyl, aryl, halo, and/or cyano;
where aryl represents a carbocyclic aromatic radical having 6 or 10 carbon atoms, which can optionally be mono-, di-, or tri-substituted, independently, with hydroxy, thiol, amino, halo, nitro, C₁₋₄-alkyl, C₁₋₄-alkylthio, C₁₋₄-alkylthio, C₁₋₄-alkyloxy, mono-C₁₋₄-alkylamino, di-C₁₋₄-alkylamino, carboxy, C₁₋₄alkoxycarbonyl, hydroxysulfonyl, C₁₋₄-alkoxysulfonyl, C₁₋₄-alkylsulfonyl, C₁₋₄-alkylsulfinyl, trifluoromethyl, cyano, tetrazolyl, carbamoyl, C₁₋₄-alkyl-carbamoyl or di-C₁₋₄-alkylcarbamoyl;
where heterocycle represents a saturated, unsaturated or aromatic cyclic radical having at least one heteroatom, selected from nitrogen, oxygen or sulfur and is optionally substituted, independently, with hydroxy, amino, imino, C₁₋₄-alkyl, C₁₋₄-alkoxy, carboxy, C₁₋₄-alkoxycarbonyl, carbamoyl, C₁₋₄-acyl, aryl, halo, and/or cyano;
and
R² is phenyl, unsubstituted or independently mono-, di-, tri-, or tetra-substituted with C₁₋₄-alkyl, hydroxy, thiol, amino, halo, nitro, cyano, C₁₋₄-alkoxy, C₁₋₄-alkylthio, mono- or di-C₁₋₄-alkylamino, carboxy, C₁₋₄-alkoxycarbonyl, C₁₋₄-alkylene-dioxy, hydroxysulfonyl, C₁₋₄-alkoxysulfbnyl, C₁₋₄-alkylsulfonyl, C₁₋₄-alkylsulfinyl, trifluoromethyl, trifluoromethyloxy, tetrazolyl, carbamoyl, and mono-or di-C₁₋₄-alkyl carbamoyl; provided that when R¹ is methoxy, R² is not 4-aminophenyl or 4-nitrophenyl;
or pharmaceutically acceptable salts or N-oxides thereof, in the manufacture of a medicament for treating asthma, allergy, rhinitis, atopic dermatitis, pain, inflammatory disease, allograft rejection, graft-vs-host rejection, and autoimmune disease in mammals.

12. A process for the preparation of a compound as defined in Claim 1 which comprises
(a) reacting a compound of the formula (4) 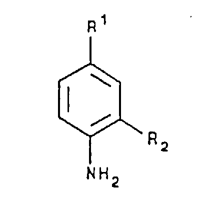 where R¹ and R² are as defined in Claim 1, with glycerol in the presence of an oxidant; or
(b) reacting a compound of formula (5) 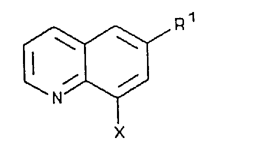 where R¹ is as defined in Claim 1 and X is chloro, bromo or iodo, with a boronic acid of the formula (3B)
R²-B(OH)₂
where R² is as defined in Claim 1; or
(c) reacting a compound of the formula 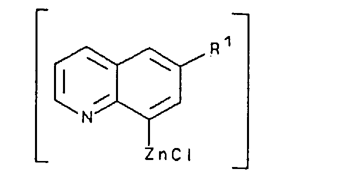 where R¹ is as defined in Claim 1, with a trifluoromethanesulfonyloxy benzene of the formula (3C)
R²-OTf
where R² is as defined in Claim 1 and -OTfis trifluoromethanesulfonyloxy; or
(d) reacting a compound of formula (5) 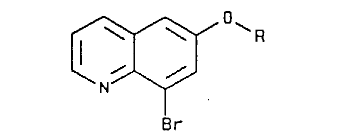 where R is C₁₋₄-alkyl; C₃₋₆-cycloalkyl; aryl or heterocycle as defined in Claim 1, with a boronic acid of the formula (3B)
R²-B(OH)₂
where R² is as defined in Claim 1; or
(e) reacting a compound of the formula (I) 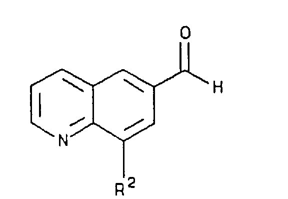 where R² is as defined in Claim 1, with a C₁₋₄-alkyl-Grignard reagent or a C₁₋₄-alkyl lithium reagent; or
(f) reacting a compound of the formula 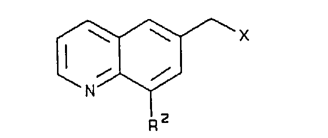 where R² is as defined in Claim 1, with a boronic acid of the formula (3B)
R-B(OH)₂
where R is C₃₋₆-cycloalkenyl; aryl or heterocycle as defined in Claim 1; or
(g) reacting a compound of the formula 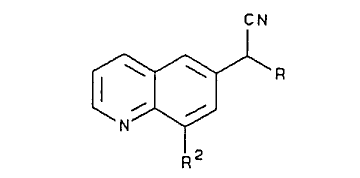 where R² is as defined in Claim 1, and R is C₁₋₄-alkyl, C₃₋₆-cycloalkyl, or heterocycle as defined in Claim 1, with a concentrated acid; or
(h) reacting a compound of the formula 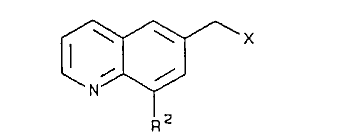 where R² is as defined in Claim 1, and X is chloro, bromo or iodo, with a compound of the formula
NH₂R or NHR₂,
where R is C₁₋₄-alkyl; C₃₋₆-cycloalkyl; aryl or heterocyle as define in Claim 1; and R² is independently C₁₋₄-alkyl; or aryl as defined in Claim 1; or NR₂ is heterocycle as defined in Claim 1; or
(i) reacting the free base of a compound of Formula (I) as defined in Claim 1 with an acid to give a pharmaceutically acceptable acid addition salt; or
(j) reacting an acid addition salt of a compound of Formula (I) as defined in Claim 1 with a base to give the corresponding free base;
(k) converting an acid addition salt of a compound of Formula (I) as defined in Claim 1 to another pharmaceutically acceptable acid addition salt of Formula (I) as defined in Claim 1.

## Patentansprüche

1. Verbindung der Formel: 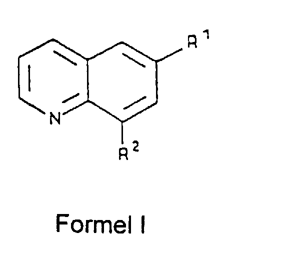 worin:
R¹ ausgewählt ist aus C₁₋₄-Alkyl, C₃₋₆-Cydoalkyl_{,} C₃₋₆-Cydoalkyloxy_{,} C₃₋₆-Cyclo-alkylamino, C₃₋₆-Cycloalkyl-C₁₋₄-alkyl, C₁₋₄-Alkoxy, Hydroxy-C₁₋₄-alkyl, Carboxy-C₁₋₄-alkyl, Aryl, Aryloxy, Arylamino, Aryl-C₁₋₄alkyl, Heterocyclus, Heterocyclusoxy, Heterocyclus-amino und Heterocyclus-C₁₋₄-alkyl,
wobei Cycloalkyl gegebenenfalls substituiert ist mit Hydroxy, Amino, Imino, C₁₋₄-Alkyl, C₁₋₄-Alkoxy, Carboxy, C₁₋₄-Alkoxycarbonyl, Carbamoyl, C₁₋₄-Acyl, Aryl, Halogen und/oder Cyano;
wobei Aryl für einen carbocyclischen aromatischen Rest mit 6 oder 10 Kohlenstoffatomen steht, der gegebenenfalls unabhängig mono-, di- oder trisubstituiert sein kann mit Hydroxy, Thiol, Amino, Halogen, Nitro, C₁₋₄-Alkyl, C₁₋₄-Alkylthio, C₁₋₄-Alkyloxy, Mono-C₁₋₄-alkylamino, Di-C₁₋₄-alkylamino, Carboxy, C₁₋₄-Alkoxycarbonyl, Hydroxysulfonyl, C₁₋₄-Alkoxysulfonyl, C₁₋₄-Alkylsulfonyl, C₁₋₄-Alkylsulfinyl, Trifluormethyl, Cyano, Tetrazolyl, Carbamoyl, C₁₋₄-Alkylcarbamoyl oder Di-C₁₋₄-alkylcarbamoyl;
wobei Heterocyclus für einen gesättigten, ungesättigten oder aromatischen cyclischen Rest mit mindestens einem Heteroatom, ausgewählt aus Stickstoff, Sauerstoff oder Schwefel, steht und gegebenenfalls unabhängig substituiert ist
mit Hydroxy, Amino, Imino, C₁₋₄-Alkyl, C₁₋₄-Alkoxy, Carboxy, C₁₋₄-Alkoxycarbonyl, Carbamoyl, C₁₋₄-Acyl, Aryl, Halogen und/oder Cyano; und
R² steht für Phenyl, unsubstituiert oder unabhängig mono-, di-, tri- oder tetra-substituiert mit C₁₋₄-Alkyl, Hydroxy, Thiol, Amino, Halogen, Nitro, Cyano, C₁₋₄-Alkoxy, C₁₋₄-Alkylthio, Mono- oder Di-C₁₋₄-alkylamino, Carboxy, C₁₋₄-Alkoxycarbonyl, C₁₋₄-Alkylendioxy, Hydroxysulfonyl, C₁₋₄-Alkoxysulfonyl, C₁₋₄-Alkylsulfonyl, C₁₋₄-Alkylsulfinyl, Trifluormethyl, Trifluormethyloxy, Tetrazolyl, Carbamoyl und Mono- oder Di-C₁₋₄-alkylcarbamoyl;
mit der Maßgabe, daß wenn R¹ für Methoxy steht, R² nicht 4-Aminophenyl oder 4-Nitrophenyl ist;
oder pharmazeutisch annehmbare Salze oder N-Oxide derselben.

2. Verbindung nach Anspruch 1, in welcher R² für Phenyl, das in der 3-Stellung mit Halogen, Nitro, Cyano, C₁₋₄-Alkoxycarbonyl oder Trifluormethyl substituiert ist, steht.

3. Verbindung nach Anspruch 2, in welcher R² 3-Chlorphenyl, 3-Nitrophenyl oder 3-Cyanophenyl darstellt.

4. Verbindung nach Anspruch 3, in welcher R¹ Pyridylmethyl, Benzyl, C₁₋₄-Alkyl, Hydroxy-C₁₋₄-alkyl oder C₃₋₆-Cydoalkylmethyl darstellt.

5. Verbindung nach Anspruch 4, in welcher R¹ 4-Pyridylmethyl, Benzyl, Ethyl, n-Propyl, Isopropyl, n-Butyl, 1-Hydroxy-1-methylethyl, Cyclopropylmethyl oder Cyclopentylmethyl ist.

6. Verbindung nach Anspruch 1, in welcher R² für in der 3- und 4-Stellung unabhängig mit Halogen, Nitro, Cyano, C₁₋₄-Alkoxycarbonyl, C₁₋₄-Alkylendioxy oder Trifluormethyl substituiertes Phenyl steht.

7. Verbindung nach Anspruch 6, in welcher R² 3-Chlor-4-fluorphenyl oder 3,4-Methylendioxyphenyl darstellt.

8. Verbindung nach Anspruch 7, in welcher R¹ Pyridylmethyl, Benzyl, C₁₋₄-Alkyl, Hydroxy-C₁₋₄-alkyl oder C₃₋₆-Cycloalkylmethyl darstellt.

9. Verbindung nach Anspruch 8, in welcher R¹ 4-Pyridylmethyl, Benzyl, Ethyl, n-Propyl, Isopropyl, n-Butyl, 1-Hydroxy-1-methylethyl, Cyclopropylmethyl oder Cyclopentylmethyl bedeutet.

10. Pharmazeutische Zusammensetzung, umfassend einen pharmazeutisch annehmbaren Exzipienten und eine therapeutisch wirksame Menge einer Verbindung wie in Anspruch 1 definiert.

11. Verwendung einer Verbindung, die dargestellt wird durch die Formel 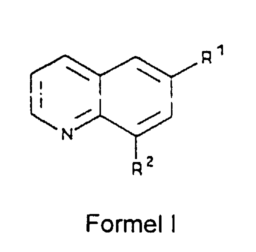 worin:
R¹ ausgewählt ist aus Wasserstoff, C₁₋₄-Alkyl, C₃₋₆-Cycloalkyl, C₃₋₆-Cydoalkyloxy, C₃₋₆-Cydoalkylamino, C₃₋₆-Cycloalkyl-C₁₋₄-alkyl, C₁₋₄-Alkoxy, Formyl, Hydroxy-C₁₋₄-alkyl, Carboxy-C₁₋₄-alkyl, Aryl, Aryloxy, Arylamino, Aryl-C₁₋₄-alkyl, Heterocyclus, Heterocyclusoxy, Heterocyclus-amino und Heterocydus-C₁₋₄-alkyl,
wobei Cycloalkyl gegebenenfalls substituiert ist mit Hydroxy, Amino, Imino, C₁₋₄-Alkyl, C₁₋₄-Alkoxy, Carboxy, C₁₋₄-Alkoxycarbonyl, Carbamoyl, C₁₋₄-Acyl, Aryl, Halogen und/oder Cyano;
wobei Aryl für einen carbocyclischen aromatischen Rest mit 6 oder 10 Kohlenstoffatomen steht, der gegebenenfalls unabhängig mono-, di- oder trisubstituiert sein kann mit Hydroxy, Thiol, Amino, Halogen, Nitro, C₁₋₄-Alkyl, C₁₋₄-Alkylthio, C₁₋₄-Alkyloxy, Mono-C₁₋₄-alkylamino, Di-C₁₋₄-alkylamino, Carboxy, C₁₋₄-Alkoxycarbonyl, Hydroxysulfonyl, C₁₋₄-Alkoxysulfonyl, C₁₋₄-Alkylsulfonyl, C₁₋₄-Alkylsulfinyl, Trifluormethyl, Cyano, Tetrazolyl, Carbamoyl, C₁₋₄-Alkylcarbamoyl oder Di-C₁₋₄-alkylcarbamoyl;
wobei Heterocyclus für einen gesättigten, ungesättigten oder aromatischen cyclischen Rest mit mindestens einem Heteroatom, ausgewählt aus Stickstoff, Sauerstoff oder Schwefel, steht und gegebenenfalls unabhängig substituiert ist
mit Hydroxy, Amino, Imino, C₁₋₄-Alkyl, C₁₋₄-Alkoxy, Carboxy, C₁₋₄-Alkoxycarbonyl, Carbamoyl, C₁₋₄-Acyl, Aryl, Halogen und/oder Cyano; und
R² steht für Phenyl, unsubstituiert oder unabhängig mono-, di-, tri- oder tetra-substituiert mit C₁₋₄-Alkyl, Hydroxy, Thiol, Amino, Halogen, Nitro, Cyano, C₁₋₄-Alkoxy, C₁₋₄-Alkylthio, Mono- oder Di-C₁₋₄-alkylamino, Carboxy, C₁₋₄-Alkoxycarbonyl, C₁₋₄-Alkylendioxy, Hydroxysulfonyl, C₁₋₄-Alkoxysulfonyl, C₁₋₄-Alkylsulfonyl, C₁₋₄-Alkylsulfinyl, Trifluormethyl, Trifluormethyloxy, Tetrazolyl, Carbamoyl und Mono- oder Di-C₁₋₄-alkylcarbamoyl;
mit der Maßgabe, daß wenn R¹ für Methoxy steht, R² nicht 4-Aminophenyl oder 4-Nitrophenyl ist;
oder pharmazeutisch annehmbarer Salze oder N-Oxide derselben bei der Herstellung eines Medikaments für die Behandlung von Asthma, Allergie, Rhinitis, atopischer Dermatitis, Schmerz, Entzündungskrankheit, Fremdtransplantat-Abstoßung, Transplantat-gegen-Wirt-Abstoßung und Autoimmunkrankheit in Säugern.

12. Verfahren zur Herstellung einer Verbindung nach Anspruch 1, umfassend
(a) Umsetzung einer Verbindung der Formel (4) 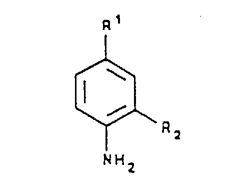 worin R¹ und R² wie in Anspruch 1 definiert sind, mit Glycerin in Anwesenheit eines Oxidationsmittels; oder
(b) Umsetzung einer Verbindung der Formel (5) 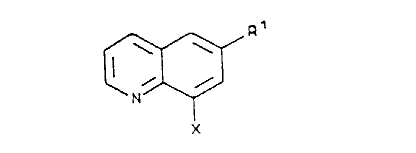 worin R¹ wie in Anspruch 1 definiert ist und X für Chlor, Brom oder lod steht, mit einer Boronsäure der Formel (3B)
R²-B(OH)₂
worin R² wie in Anspruch 1 definiert ist; oder
(c) Umsetzung einer Verbindung der Formel 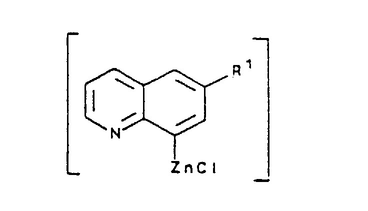 worin R¹ wie in Anspruch 1 definiert ist, mit einem Trifluormethansulfonyloxybenzol der Formel (3C)
R²-OTf
worin R² wie in Anspruch 1 definiert ist und -OTf für Trifluormethansulfonyloxy steht; oder
(d) Umsetzung einer Verbindung der Formel (5) 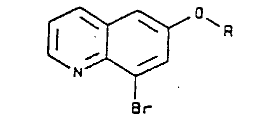 worin R C₁₋₄-Alkyl; C₃₋₆-Cycloalkyl; Aryl oder Heterocyclus wie in Anspruch 1 definiert bedeutet, mit einer Boronsäure der Formel (3B)
R²-B(OH)₂
worin R² wie in Anspruch 1 definiert ist; oder
(e) Umsetzung einer Verbindung der Formel (I) 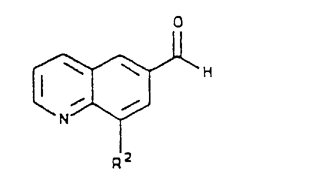 worin R² wie in Anspruch 1 definiert ist, mit einem C₁₋₄-Alkyl-Grignard-Reagenz oder einem C₁₋₄-Alkyllithium-Reagenz; oder
(f) Umsetzung einer Verbindung der Formel 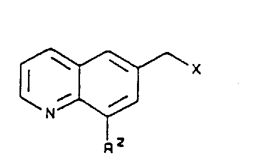 worin R² wie in Anspruch 1 definiert ist, mit einer Boronsäure der Formel (3B)
R-B(OH)₂
worin R für C₃₋₆-Cycloalkenyl; Aryl oder Heterocyclus wie in Anspruch 1 definiert steht; oder
(g) Umsetzung einer Verbindung der Formel 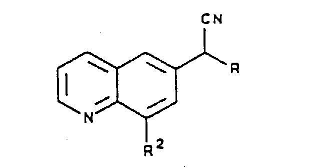 worin R² wie in Anspruch 1 definiert ist und R für C₁₋₄-Alkyl, C₃₋₆-Cycloalkyl oder Heterocyclus wie in Anspruch 1 definiert steht, mit einer konzentrierten Säure; oder
(h) Umsetzung einer Verbindung der Formel 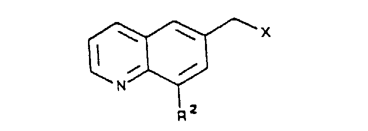 worin R² wie in Anspruch 1 definiert ist und X Chlor, Brom oder lod ist, mit einer Verbindung der Formel
NH₂R oderNHR₂
worin R für C₁₋₄-Alkyl; C₃₋₆-Cycloalkyl; Aryl oder Heterocyclus wie in Anspruch 1 definiert steht; und R₂ unabhängig C₁₋₄-Alkyl; oder Aryl wie in Anspruch 1 definiert bedeutet; oder NR₂ Heterocyclus wie in Anspruch 1 definiert darstellt; oder
(i) Umsetzung der freien Base einer Verbindung der Formel (I) wie in Anspruch 1 definiert mit einer Säure, um ein pharmazeutisch annehmbares Säureadditionssalz zu liefern; oder
(j) Umsetzung eines Säureadditionssalzes einer Verbindung der Formel (I) wie in Anspruch 1 definiert mit einer Base, um die entsprechende freie Base zu liefern;
(k) Umwandlung eines Säureadditionssalzes einer Verbindung der Formel (I) wie in Anspruch 1 definiert in ein anderes pharmazeutisch annehmbares Säureadditionssalz der Formel (I) wie in Anspruch 1 definiert.

## Revendications

1. Composé de formule 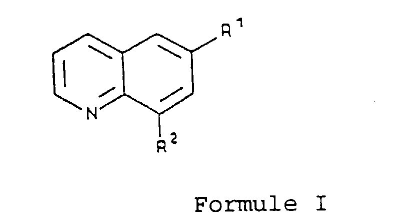 dans laquelle :
R¹ est choisi entre des groupes alkyle en C₁ à C₄, cycloalkyle en C₃ à C₆, cycloalkyloxy en C₃ à C₆, cycloalkylamino en C₃ à C₆, (cycloalkyle en C₃ à C₆) - (alkyle en C₁ à C₄), alkoxy en C₁ à C₄, hydroxy-(alkyle en C₁ à C₄), carboxy-(alkyle en C₁ à C₄), aryle, aryloxy, arylamino, aryl-(alkyle en C₁ à C₄), hétérocycle, hétérocycle-oxy, hétérocycle-amine et hétérocycle-(alkyle en C₁ à C₄)
où un groupe cycloalkyle est facultativement substitué avec un radical hydroxy, amino, imino, alkyle en C₁ à C₄, alkoxy en C₁ à C₄, carboxy, (alkoxy en C₁ à C₄)carbonyle, carbamoyle, acyle en C₁ à C₄, aryle, halogéno et/ou cyano ;
où aryle représente un radical aromatique carbocyclique ayant 6 ou 10 atomes de carbone, qui peut facultativement être mono-substitué, disubstitué ou trisubstitué, indépendamment, avec un substituant hydroxy, thiol, amino, halogéno, nitro, alkyle en C₁ à C₄, alkylthio en C₁ à C₄, alkyloxy en C₁ à C₄, mono-(alkyle en C₁ à C₄)-amine, di-(alkyle en C₁ à C₄)-amino, carboxy, (alkoxy en C₁ à C₄)-carbonyle, hydroxysulfonyle, alkoxysulfonyle en C₁ à C₄, alkylsulfonyle en C₁ à C₄, alkylsulfinyle en C₁ à C₄, trifluorométhyle, cyano, tétrazolyle, carbamoyle, (alkyle en C₁ à C₄)-carbamoyle ou di-(alkyle en C₁ à C₄)-carbamoyle ;
où hétérocycle représente un radical cyclique saturé, non saturé ou aromatique ayant au moins un hétéroatome choisi entre azote, oxygène ou soufre et est facultativement substitué, indépendamment, avec des substituants hydroxy, amino, imino, alkyle en C₁ à C₄, alkoxy en C₁ à C₄, carboxy, (alkoxy en C₁ à C₄)carbonyle, carbamoyle, acyle en C₁ à C₄, aryle, halogéno et/ou cyano ; et
R² est un groupe phényle, non substitué ou mono-substitué, disubstitué, trisubstitué ou tétra-substitué indépendamment avec des substituants alkyle en C₁ à C₄, hydroxy, thiol, amino, halogéno, nitro, cyano, alkoxy en C₁ à C₄, alkylthio en C₁ à C₄, mono- ou di-(alkyle en C₁ à C₄)-amino, carboxy, (alkoxy en C₁ à C₄)carbonyle, alkylènedioxy en C₁ à C₄, hydroxysulfonyle, alkoxysulfonyle en C₁ à C₄, alkylsulfonyle en C₁ à C₄, alkylsulfinyle en C₁ à C₄, trifluorométhyle, trifluorométhyloxy, tétrazolyle, carbamoyle et mono- ou di-(alkyle en C₁ à C₄)-carbamoyle ;
sous réserve que lorsque R¹ est un groupe méthoxy, R² ne soit pas un groupe 4-aminophényle ou 4-nitrophényle ;
ou des sels acceptables du point de vue pharmaceutique ou des N-oxydes de ce composé.

2. Composé suivant la revendication 1, dans lequel R² est un groupe phényle substitué en position 3 avec un radical halogéno, nitro, cyano, (alkoxy en C₁ à C₄)-carbonyle ou trifluorométhyle.

3. Composé suivant la revendication 2, dans lequel R² est un groupe 3-chlorophényle, 3-nitrophényle ou 3-cyanophényle.

4. Composé suivant la revendication 3, dans lequel R¹ est un groupe pyridylméthyle, benzyle, alkyle en C₁ à C₄, hydroxy-(alkyle en C₁ à C₄) ou (cycloalkyle en C₃ à C₆)-méthyle.

5. Composé suivant la revendication 4, dans lequel R¹ est un groupe 4-pyridylméthyle, benzyle, éthyle, n-propyle, isopropyle, n-butyle, 1-hydroxy-1-méthyléthyle, cyclopropylméthyle ou cyclopentylméthyle.

6. Composé suivant la revendication 1, dans lequel R² est un groupe phényle substitué en position 3 ou 4, indépendamment, avec un radical halogéno, nitro, cyano, (alkoxy en C₁ à C₄)carbonyle, alkylènedioxy en C₁ à C₄ ou trifluorométhyle.

7. Composé suivant la revendication 6, dans lequel R² est un groupe 3-chloro-4-fluorophényle ou 3,4-méthylènedioxyphényle.

8. Composé suivant la revendication 7, dans lequel R¹ est un groupe pyridylméthyle, benzyle, alkyle en C₁ à C₄, hydroxyalkyle en C₁ à C₄ ou (cycloalkyle en C₃ à C₆)méthyle.

9. Composé suivant la revendication 8, dans lequel R¹ est un groupe 4-pyridylméthyle, benzyle, éthyle, n-propyle, isopropyle, n-butyle, 1-hydroxy-1-méthyléthyle, cyclopropylméthyle ou cyclopentylméthyle.

10. Composition pharmaceutique comprenant un excipient acceptable du point de vue pharmaceutique et une quantité thérapeutiquement efficace d'un composé tel que défini dans la revendication 1.

11. Utilisation d'un composé représenté par la formule 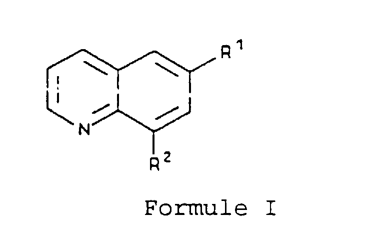 dans laquelle :
R¹ est choisi entre l'hydrogène, des groupes alkyle en C₁ à C₄, cycloalkyle en C₃ à C₆, cycloalkyloxy en C₃ à C₆, cycloalkylamino en C₃ à C₆, (cycloalkyle en C₃ à C₆) - (alkyle en C₁ à C₄), alkoxy en C₁ à C₄, formyle, hydroxy-(alkyle en C₁ à C₄), carboxy-(alkyle en C₁ à C₄), aryle, aryloxy, arylamino, aryl-(alkyle en C₁ à C₄), hétérocycle, hétérocycle-oxy, hétérocycle-amino et hétérocycle-(alkyle en C₁ à C₄)
où un groupe cycloalkyle est facultativement substitué avec un radical hydroxy, amino, imino, alkyle en C₁ à C₄, alkoxy en C₁ à C₄, carboxy, (alkoxy en C₁ à C₄)carbonyle, carbamoyle, acyle en C₁ à C₄, aryle, halogéno et/ou cyano ;
où aryle représente un radical aromatique carbocyclique ayant 6 ou 10 atomes de carbone, qui peut facultativement être mono-substitué, disubstitué ou trisubstitué, indépendamment, avec un substituant hydroxy, thiol, amino, halogéno, nitro, alkyle en C₁ à C₄, alkylthio en C₁ à C₄, alkyloxy en C₁ à C₄, mono-(alkyle en C₁ à C₄)-amino, di-(alkyle en C₁ à C₄)-amino, carboxy, (alkoxy en C₁ à C₄)-carbonyle, hydroxysulfonyle, alkoxysulfonyle en C₁ à C₄, alkylsulfonyle en C₁ à C₄, alkylsulfinyle en C₁ à C₄, trifluorométhyle, cyano, tétrazolyle, carbamoyle, (alkyle en C₁ à C₄)-carbamoyle ou di-(alkyle en C₁ à C₄)-carbamoyle ;
où hétérocycle représente un radical cyclique saturé, non saturé ou aromatique ayant au moins un hétéroatome choisi entre azote, oxygène ou soufre et est facultativement substitué, indépendamment, avec des substituants hydroxy, amino, imino, alkyle en C₁ à C₄, alkoxy en C₁ à C₄, carboxy, (alkoxy en C₁ à C₄)carbonyle, carbamoyle, acyle en C₁ à C₄, aryle, halogéno et/ou cyano ; et
R² est un groupe phényle, non substitué ou mono-substitué, disubstitué, trisubstitué ou tétra-substitué indépendamment avec des substituants alkyle en C₁ à C₄, hydroxy, thiol, amino, halogéno, nitro, cyano, alkoxy en C₁ à C₄, alkylthio en C₁ à C₄, mono- ou di-(alkyle en C₁ à C₄)-amino, carboxy, (alkoxy en C₁ à C₄)carbonyle, alkylènedioxy en C₁ à C₄, hydroxysulfonyle, alkoxysulfonyle en C₁ à C₄, alkylsulfonyle en C₁ à C₄, alkylsulfinyle en C₁ à C₄, trifluorométhyle, trifluorométhyloxy, tétrazolyle, carbamoyle et mono- ou di-(alkyle en C₁ à C₄)-carbamoyle ;
sous réserve que lorsque R¹ est un groupe méthoxy, R² ne soit pas un groupe 4-aminophényle ou 4-nitrophényle ;
ou de ses sels acceptables du point de vue pharmaceutique ou de ses N-oxydes, dans la préparation d'un médicament destiné au traitement de l'asthme, de l'allergie, de la rhinite, de la dermatite atopique, de la douleur, d'une maladie inflammatoire, d'un rejet d'allogreffe, d'une réaction de rejet du greffon contre l'hôte et d'une maladie auto-immune chez des mammifères.

12. Procédé de production d'un composé tel que défini dans la revendication 1, qui comprend :
(a) la réaction d'un composé de formule (4) 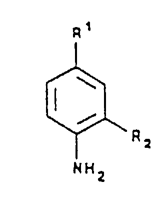 dans laquelle R¹ et R² sont tels que définis dans la revendication 1, avec le glycérol en présence d'un oxydant ; ou
(b) la réaction d'un composé de formule (5) 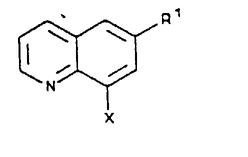 dans laquelle R¹ est tel que défini dans la revendication 1 et X est un radical chloro, bromo ou iodo, avec un acide boronique de formule (3B)
R²-B(OH)₂·
dans laquelle R² est tel que défini dans la revendication 1 ; ou
(c) la réaction d'un composé de formule 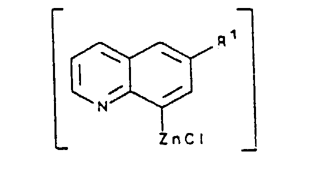 dans laquelle R¹ est tel que défini dans la revendication 1, avec un trifluorométhanesulfonyloxybenzène de formule (3C)
R²-OTf
dans laquelle R² est tel que défini dans la revendication 1 et -OTf est le groupe trifluorométhanesulfonyloxy ; ou
(d) la réaction d'un composé de fromule (5) 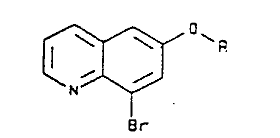 dans laquelle R est un groupe alkyle en C₁ à C₄ ; cycloalkyle en C₃ à C₆ ; aryle ou un hétérocycle tel que défini dans la revendication 1,
avec un acide boronique de formule (3B)
R²-B(OH)₂
dans laquelle R² est tel que défini dans la revendication 1 ; ou
(e) la réaction d'un composé de formule (I) 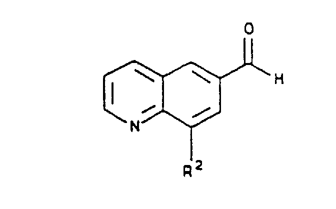 dans laquelle R² est tel que défini dans la revendication 1, avec un réactif de Grignard à groupe alkyle en C₁ à C₄ ou un réactif (alkyle en C₁ à C₄)-lithium; ou
(f) la réaction d'un composé de formule 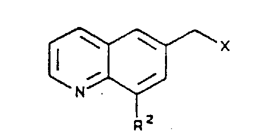 dans laquelle R² est tel que défini dans la revendication 1, avec un acide boronique de formule (3B)
R-B(OH)₂
dans laquelle R est un groupe cycloalcényle en C₃ à C₆ ; aryle ou hétérocycle tel que défini dans la revendication 1 ; ou
(g) la réaction d'un composé de formule 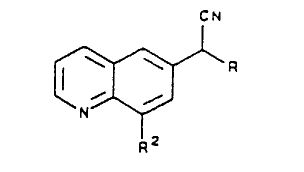 dans laquelle R² est tel que défini dans la revendication 1 et R est un groupe alkyle en C₁ à C₄, cycloalkyle en C₃ à C₆ ou hétérocycle tel que défini dans la revendication 1, avec un acide concentré ; ou
(h) la réaction d'un composé de formule 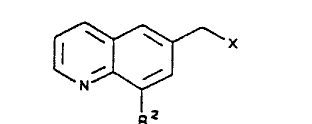 dans laquelle R² est tel que défini dans la revendication 1 et X est un radical chloro, bromo ou iodo, avec un composé de formule
NH₂R ou NHR₂,
dans laquelle R est un groupe alkyle en C₁ à C₄ ; cycloalkyle en C₃ à C₆ ; aryle ou hétérocycle tel que défini dans la revendication 1 ; et R₂ représente indépendamment un groupe alkyle en C₁ à C₄ ; ou un groupe aryle tel que défini dans la revendication 1 ; ou bien NR₂ est un hétérocycle tel que défini dans la revendication 1 ; ou
(i) la réaction de la base libre d'un composé de formule (I) tel que défini dans la revendication 1 avec un acide pour former un sel d'addition d'acide acceptable du point de vue pharmaceutique ; ou
(j) la réaction d'un sel d'addition d'acide d'un composé de formule (I) tel que défini dans la revendication 1 avec une base pour former la base libre correspondante ;
(k) la transformation d'un sel d'addition d'acide d'un composé de formule (I) tel que défini dans la revendication 1 en un autre sel d'addition d'acide acceptable du point de vue pharmaceutique de formule (I) tel que défini dans la revendication 1.
